# EUROPEAN PATENT SPECIFICATION

(11) **EP 1 208 230 B1**
(45) Date of publication and mention of the grant of the patent: **30.11.2005**
(21) Application number: 00936714.5
(22) Date of filing: 05.05.2000
(51) Int. Cl.: C12Q 1/68

(54) **HIGH DENSITY LABELING OF DNA WITH MODIFIED OR "CHROMOPHORE"CARRYING NUCLEOTIDES AND DNA POLYMERASES USED**
HOHE DICHTE MARKIERUNG VON DNS MIT MODIFIZIERTE ODER CHROMOPHORE TRAGENDE NUKLEOTIDE UND BENUTZTE DNS POLYMERASEN
MARQUAGE HAUTE DENSITE D'ADN PAR DES NUCLEOTIDES MODIFIES OU PORTANT UN CHROMATOPHORE ET ADN POLYMERASES MISES EN APPLICATION

(30) Priority: 07.05.1999 EP 99108601
(43) Date of publication of application: 29.05.2002
(73) Proprietor: Roche Diagnostics GmbH, 68305 Mannheim (DE)
(72) Inventor: MUEHLEGGER, Klaus, D-82398 Polling (DE); ANGERER, Bernhard, D-83024 Rosenheim (DE); SEELA, Frank, D-49076 Osnabrueck (DE); ANKENBAUER, Waltraud, D-82377 Penzberg (DE); AUGUSTIN, Martin, D-82152 Planegg (DE); GUMBIOWSKI, Karin, D-49134 Wallenhorst (DE); ZULAUF, Matthias, D-49088 Osnabrueck (DE)
(86) International application number: PCT/EP2000/004036
(87) International publication number: WO 2000/068422

(56) References cited:
- WO-A-92/00989
- WO-A-92/06216
- WO-A-93/03180
- DATABASE BIOSIS [Online] BIOSCIENCES INFORMATION SERVICE, PHILADELPHIA, PA, US; 1994 KOMMINOTH PAUL ET AL: "Evaluation of methods for hepatitis C virus detection in archival liver biopsies." Database accession no. PREV199598141463 XP002182194 & PATHOLOGY RESEARCH AND PRACTICE, vol. 190, no. 11, 1994, pages 1017-1025, ISSN: 0344-0338

## Description

Synthesis of labeled DNA via incorporation of modified deoxynucleosidetriphosphates which carry a spacer arm and a detectable group is a common method in molecular biology. Labeling of nucleic acids with modified nucleotides in high density however has not been shown so far. It was assumed that steric hindrance between the linker arms and the detectable group (reporter groups or dyes) precludes the synthesis of DNA fragments in which each base or a major fraction of these bases is linked to a detectable group.
1. DIG-labeled nucleotides can be incorporated, to a defined density into nucleic acid probes by DNA polymerases (such as E.coli DNA polymerase I, DNA polymerasesT4 or T7, reverse transcriptase, Taq polymerase or Terminal transferase) by random primed labeling, nick translation, PCR, 3'-end labeling/ tailing or in vitro transcription. The labeling mixture contains Dig-dUTP and dTTP, dATP, dCTP and dGTP. The labelled nucleotide Dig-dUTP cannot entirely replace dTTP in the initial reaction mixture because the reaction seems to be inhibited by Dig-dUTP when using the commonly used reaction components and DNA polymerases commonly used for DNA labeling. When an optimized ratio of Dig-dUTP:dTTP is used, an increase in Dig-dUTP concentration results in some higher labeling density but reduction in product length and yield of the product. It was not possible so far to replace dTTP entirely by Dig-dUTP using the common labeling techniques (Nonradioactive In Situ Hybridization Application Manual 2nd edition, Boehringer Mannheim GmbH 1996) [4].
2. Jett J. H. et al (US Patent 5,405,747 Method for rapid base sequencing in DNA and RNA with two base labeling, 1995) [5,6] describe that DNA strands up to 500 nucleotides in length containing one fluorescent nucleotide and three unmodified nucleotides have been synthesized. The DNA synthesis was performed using a mutated T7 DNA polymerase and Rhodamin-dCTP, Rhodamin-dATP, Rhodamin-dUTP, Fluorescein-dATP or Fluorescein-dUTP. DNA synthesis has also been observed by modified T4 DNA polymerase with Rhodamine-dCTP or Rhodamine-dATP. The authors do not comment on the efficiency of incorporation of modified nucleotides versus unmodified bases in immediately adjacent positions but discuss the difficulty of incorporation of the labeled nucleotides by the DNA polymerases because of steric hindrance. Replacement of more than one normal nucleoside triphosphate by derivatives at once is not described by these authors.
3. Makiko Hiyoshi & Shigeru Hosoi (Assay of DNA Denaturation by PCR-driven Fluorescent Label Incorporation and Fluorescence Resonance Energy Transfer Analytical Biochemistry (1994) 221, 306-311.) [7] describe the incorporation of fluorescent labels such as Fluorescein-11-dUTP or Rhodamine-4-dUTP during PCR amplification using Taq DNA polymerase. These fluorophore labeled nucleotides were used in mixtures containing dTTP. The authors describe that these desoxynucleoside triphosphate derivatives were unable to substitute entirely for the normal substrate, dTTP. These results were interpreted such that the steric hindrance of the linker arm and the fluorescent group inhibits specific incorporation into neighbouring positions.
4. Incorporation of Cy5-modified desoxyuridyl triphosphates with different lengths of linker arms is described in Yu H. et al. NAR (1994) 22, 3418-3422. These substrates were analyzed in nick-translation reactions performed with E.coli DNA polymerase I and in PCR using Taq DNA polymerase. The level of incorporation increased in parallel when the length of the linker arm was increased. Under optimal conditions it was possible to label up to 28 % of the possible substitution sites on the target DNA with reasonable yield by PCR and 18 % by nick translation. The failure of complete substitution was explained to be caused by steric interactions between the polymerase and the cyanine-labeled sites on the template (for PCR) and extending chains and the modified dUTP substrate.
5. Starke H. R. et al. (Nucl. Acids. Res. (1994) 22, 3997-4001) [9] describe enzymatic DNA labeling-synthesis on M13 DNA as template, Fluorescein-15-dATP or Tetramethylrhodamine-dATP, dCTP, dGTP, TTP, modified T7 DNA polymerase (Sequenase) and Mn⁺⁺ as divalent metal ion. Use of Mn⁺⁺ instead of Mg⁺⁺ promotes misincorporation of mismatched bases. Under these conditions the polymerase not necessarily incorporates the correct base. If the incorporation of the correct base is difficult, e.g. because of steric hindrance, the incorporation of a non-complementary base is prefered. The authors show, that the labeled nucleotides are incorporated to some extent. But a detailed analysis shows, that e.g. a sequence close to the primer at which four dA-nucleotides should be incorporated contiguously is not synthesized as expected. The main product (80-90%) of this labeling reaction contains only one labeled dATP and three non-complementary nucleotides. Products containing two or three labeled dATPs are observed as a minor fraction. The authors do not describe a product containing four labeled dATPs.
6. An example in which two labeled bases were successfully incorporated by DNA polymerases is the report of Davis L. M. et al. (GATA (1991), 8(1); 1-7) [10]. Using Biotin-labeled nucleotides like Bio-11-dCTP or Bio-11-dUTP and *E. coli* DNA polymerase I (Klenow large Fragment), d(A,G)₂₁₀₀ as template/primer, a string of completely biotinylated nucleotides was generated. The authors state that steric effects are not a problem with biotinylated nucleotides. The combination of Bio-11-dCTP and Bio-11-dUTP together with dATP and dGTP were tested on M13 single stranded DNA as a template containing all four bases. With this experiment it was not possible to generate full length product.
7. Goodman, M. F. and Reha-Krantz L. (International Application Number PCT/US97/06493, WO 97/39150, Synthesis of Fluorophore-labeled DNA) describe mutant bacteriophage T4 DNA polymerases which are claimed to have increased ability to incorporate modified nucleotides for the synthesis of modified, e.g., fluorophore-labeled DNA. The mutants are described to have increased intrinsic processivity relative to the native enzyme. In reactions in which one natural nucleotide is replaced by a rhodamine-labeled nucleotide the mutant DNA polymerases generate longer stretches of DNA product than the wild type enzyme. When two natural nucleoside triphosphates were completely replaced by rhodamine-labeled dNTPs the product length decreased in comparison to the reaction in which one rhodamine labeled dNTP was used, the authors do not comment on the absolute length of the products. Full length product is only described to be obtained with the L422M mutant of T4 DNA polymerase and incorporation of Rhodamine-dUTP or Rhodamine-dCTP or Biotin-dCTP.

These examples show that labeling of DNA with modified nucleotides by DNA synthesis using DNA polymerases with more than one modified nucleotides generating full length product was not achievable so far.

However, high density labeled DNA is a necessary requirement for ultrasensitive detection of specific target molecules, like nucleic acid sequences, for single molecule sequencing or for single molecule detection. Modified nucleotides like fluorophore-, DIG-, biotin-labeled nucleotides must be incorporated into DNA by primer elongation or PCR and full length products must be generated by DNA polymerases which work at higher temperatures. Therefore, the subject of the present invention is to provide a method of labeling of DNA by DNA synthesis using a DNA polymerase whereas at least two natural occurring nucleotides are completely replaced by modified nucleotides and a full length product is generated.

These and other objects are achieved by the present invention which provides methods for template-directed synthesis of DNA with DNA polymerases and modified nucleoside triphosphates (nucleotides carrying a detectable modification covalently attached to the base). The DNA polymerases incorporate the modified deoxynucleoside triphosphates in a template-directed manner into the complementary strand, in which a major fraction or all of the nucleotides contain a modification whereas at least two natural occurring nucleotides are completely replaced by modified nucleotides. The method comprises synthesis of DNA with one or more DNA polymerases and a nucleoside triphosphate mixture in which at least two bases consist of modified nucleotides exclusively, one or more DNA polymerases which are able to synthesize DNA by incorporating modified nucleotides, whereas the one or more DNA polymerases synthesize stretches of DNA containing modified nucleotides exclusively. The invention also comprises DNA polymerases which can be used in PCR reactions with a set of nucleoside triphosphates in which one or at least two bases consist of modified bases exclusively. The inventive polymerases are belonging to the class of B-type polymerases which do not exhibit essential 3'exonuclease activity. Furthermore, modified nucleotides which are attached to hydrophilic dyes or other hydrophilic labels are preferred according to the present invention.

Especially preferred are dyes selected from the group consisting of carbocyanines, rhodoamines, fluoresceins, cumarines, vitamines as e.g. biotin, haptens as e.g. digoxigenine, oxazines and hormons as e.g. cholesterole and estradiol.

When performing the method for synthesis of high-density-labeled DNA on solid phase it is also possible to use labels which are less hydrophilic as the above mentioned examples, because the hydrophilic nature of the label is not such a critical parameter. However, in case the method according to the present invention is performed in liquid phase hydrophilic labels are preferred.

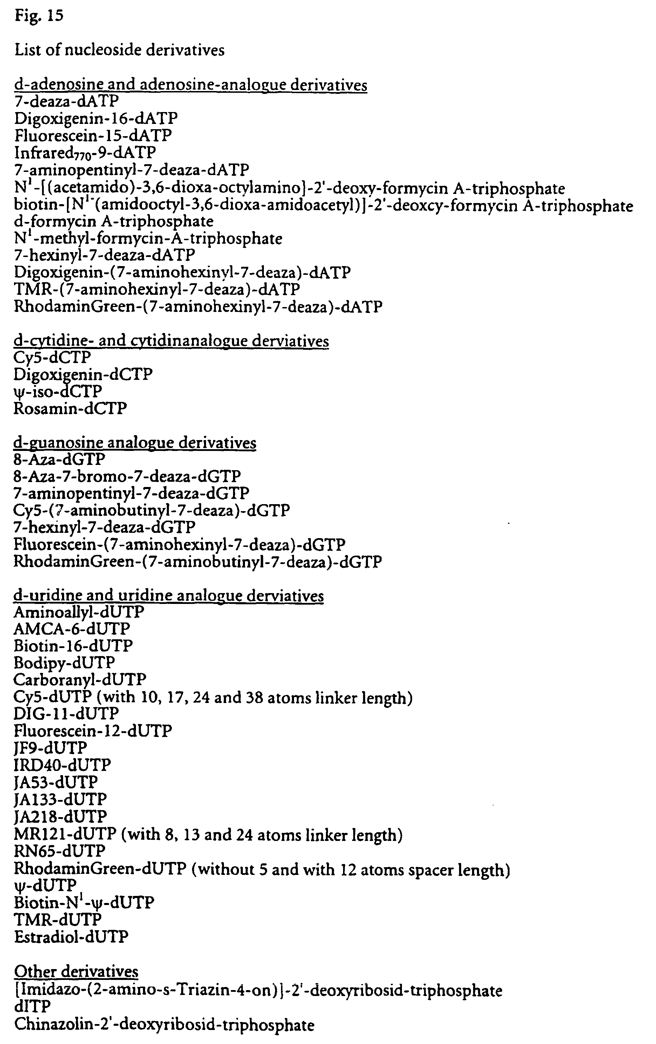

Appropriate linkers can be e.g. peptides or lipids or derivatives thereof. It is preferred that the length of the linker which attaches the dyes to the nucleotide is about 15 carbon atoms and more. Provided are examples for nucleic acid labeling using a modified nucleotide which can be detected by fluorescence (Fluorescein, Rhodamin-green or Cy5), a nucleotide which can be detected by an antibody reaction (Digoxigenin, Fluorescein), a nudeotide which can be detected by the specific interaction with Streptavidin (Biotin) and a nucleotide which carries a reactive group which can be linked to a label chemically (Aminopentinyl-C7-deaza-dATP). The length of the linker depends on the label which is attached to the nucleotide. For most labels a linker length of about 15 and more carbon atoms turned out to be favourable. However, for some smaller labels as e.g. AMCA it was favourable to use a linker containing 6 carbon atoms. Examples of nucleotide derivatives useful for the present invention are listed in Fig. 15.

In a preferred embodiment the inventive method is carried out by using a B type polymerase exhibiting no 3'exonuclese activity and nucleotides which are attached to hydrophilic labels selected from a group consisting of the above mentioned dyes whereas the linker which attaches the dye to the nucleotide has a length of about 15 carbon atoms or more.

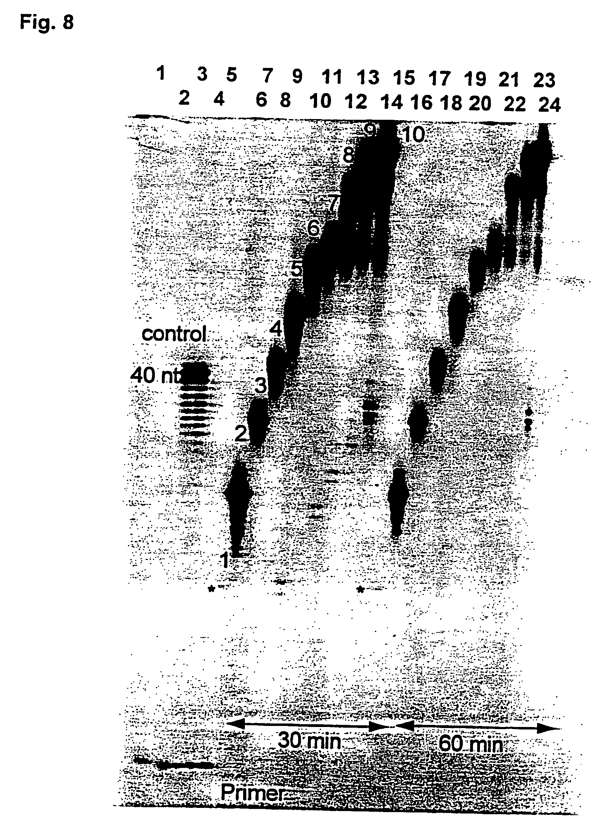

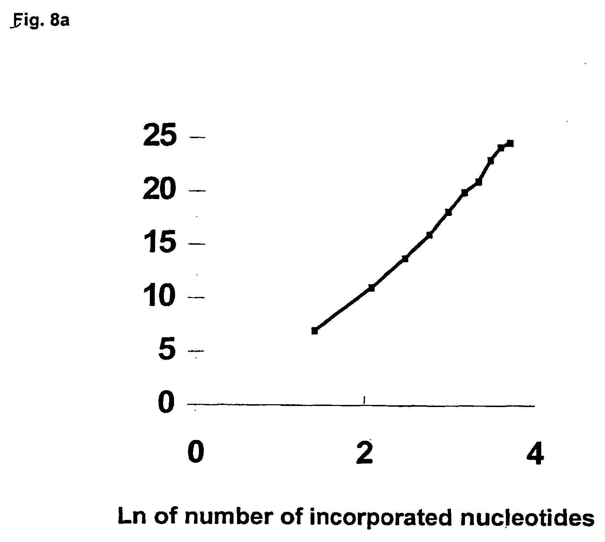

The incorporation efficiency of 11 different DNA polymerases (or blends of polymerases) and reverse transcriptases of different families [3] was studied.
1. Klenow-fragment of DNA pol I from *E. coli* (Roche Molecular Biochemicals, RMB) was chosen as an example of a well characterised mesophile A-type polymerase with known protein crystal structure.
2. Klenow-fragment of *Chy* DNA pol I from Carboxydothermus hydrogenoformans was chosen as an example of a homologue thermophilic A-type polymerase with similarity to Klenow-fragment of *E. coli.*
3. Sequenase (Amersham) is a genetically engineered version ofT7-DNA-polymerase, which is most often used for DNA-sequence analysis.
4. M-MuLV reverse transcriptase (RMB) is a single subunit enzyme and
5. AMV reverse transcriptase (RMB) an α/β subunit dimeric enzyme.
6. *Taq* polymerase (RMB) is a thermophilic A-type polymerase, also with resolved protein structure.
7. *Vent* polymerase and *Vent* exo⁻ polymerase (New England Biolabs) are thermopilic B-type polymerases. *Vent* polymerase has also additional 3'-5' exonucleolytic proof-reading activity.
8. *Tgo* (and *Tgo* exo⁻ polymerase, RMB; commercially not available, with a genetically deleted 3'-5' exonuclease function) is also a thermophilic, archaeal B-family enzyme (Example 7, Figure 8).
*9. Pwo* polymerase (RMB), is also a B-type enzyme of archaeal source. It displays additionally 3'-5' exonucleolytic proofreading activity.
10. Recombinant Pol β (rat) kindly provided by Samuel Wilson (Galveston/ Texas) to RMB.
11. Expand™ High Fidelity PCR System (RMB) is a unique blend of *Taq* and *Pwo* polymerase for superior PCR performance.

*Vent, Vent* exo⁻ and *Tgo* exo⁻ polymerase are thermopilic B-type enzyme of archaeal source and show some similarity to eucaryotic replicative α-like DNA polymerases. Wild type enzymes of these two enzymes exhibit strong 3'-5' exonuclease activity and are therefore less convenient for DNA-labeling than their engineered counterparts (exo⁻ variants) (1).

The exampels of this patent application comprise 5 polymerases or blends (nos. 2., 7., 8., 9., 11.) see no. 11 above) of the listed polymerases here, which showed superior labeling efficiencies compared with the other polymerases (data not shown in detail).

All DNA polymerases were assayed with the buffers supplied, or otherwise as indicated (*Tgo* exo⁻ was assayed in a buffer containing 50 mM Tris-HCL, pH = 8.5 at 20°C, 10 mM KCl, 15 mM (NH₄)₂SO₄, 7 mM MgSO₄, 0.05% (or 0.005% as indicated) Triton X-100 and 10 mM 2-Mercaptoethanol).

Modified deoxyribonucleotide triphosphates according to the present invention are deoxyribonucleotide triphosphates which are attached to labels. Derivatives of these modified deoxyribonucleotide triphosphates are modified additionally to this label, as e.g. modified 7-deaza-deoxyribonucleotide triphosphates or modifed C-nucleoside triphospates.

**Examples of modified Deoxyribonucleotide triphospates which are suitable for the inventive method are the following:**
**dAdenosin derivatives**
   7-Hexinyl-7-Desaza-dATP (structure see appendix 1)
   7-Aminopentinyl-7-desaza-dATP (structure see appendix 1)
**dUridin derivatives**
   AMCA-6-dUTP (RMB, Cat. No. 1534386)
   Biotin-16-dUTP (RMB, Cat. No. 1093070)
   Cy5-10-dUTP (with 10 atoms spacerlength, RMB, structures see appendix 1)
   Cy5-dUTP (with 17, 24 and 38 atoms spacerlength, RMB, structures see appendix 1)
   DIG-11-dUTP (RMB, Cat. No. 1558706)
   MR121-dUTP (with 8, 13 and 24 atoms spacerlength, RMB, structures see appendix 1)
   Fluorescein-12-dUTP (RMB, Cat. No. 1373242)
   RhodaminGreen-dUTP (RMB, structure see appendix 1)
   RhodaminGreen-X-dUTP (RMB, structure see appendix 1)
   TMR-6-dUTP (RMB, Cat. No. 1534378)
**dCytidin- derivatives**
   FluoroLink™ Cy5™-dCTP (Amersham Life Science, Cat. No. PA55021)
   Digoxigenin-28-dCTP (RMB, structure see appendix 1)
   Rosamin-dCTP (RMB, structure see appendix 1)
**dGuanosine-derivatives**
   7-Hexinyl-7-desaza-dGTP (structure see appendix 1)

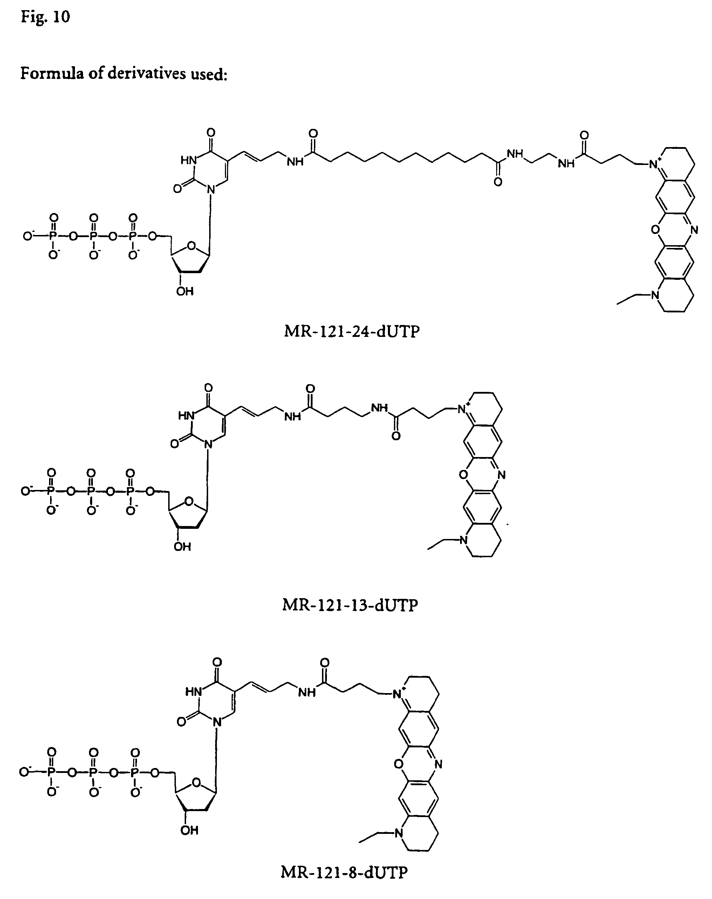

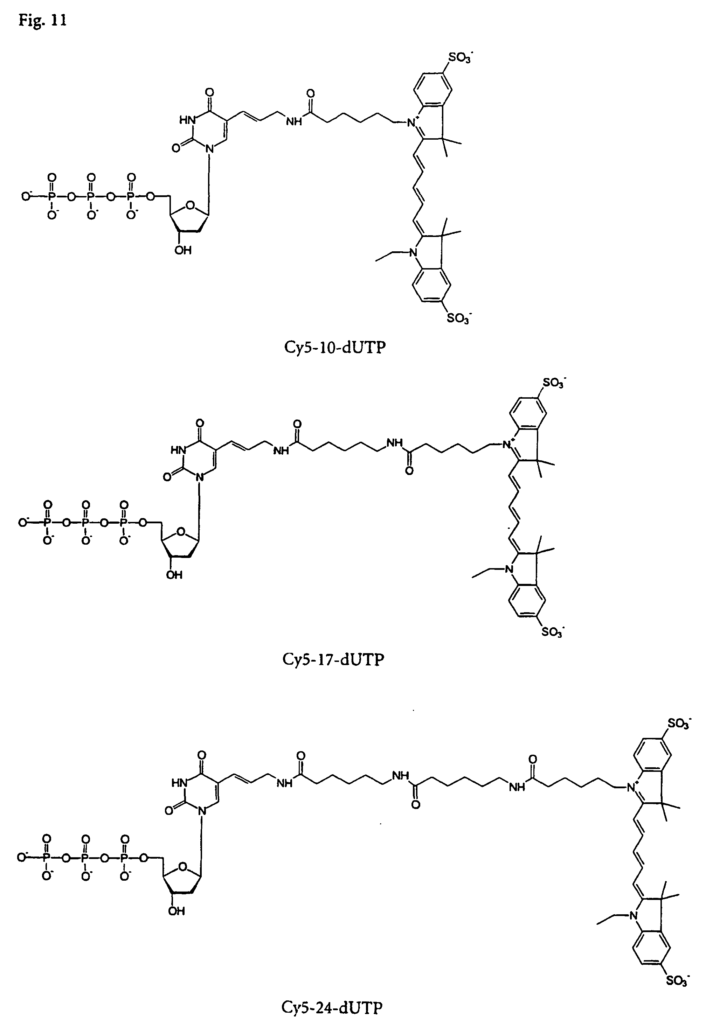

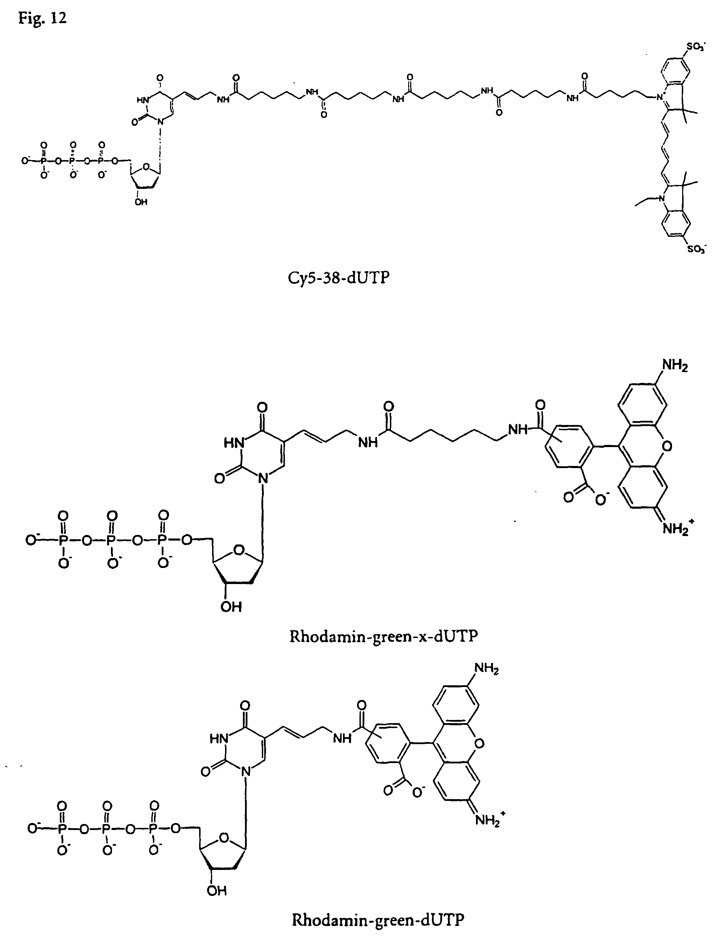

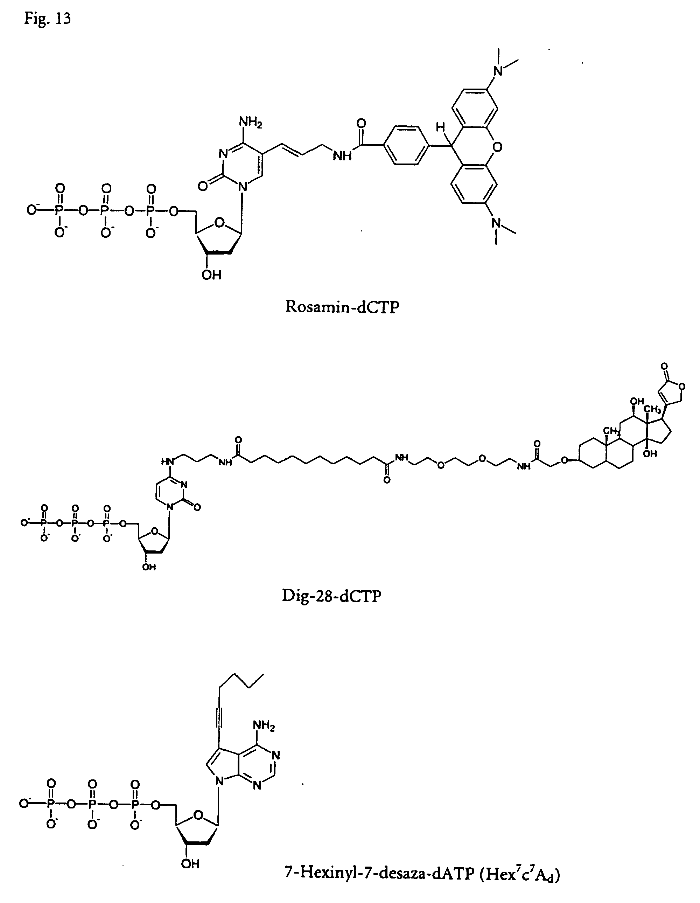

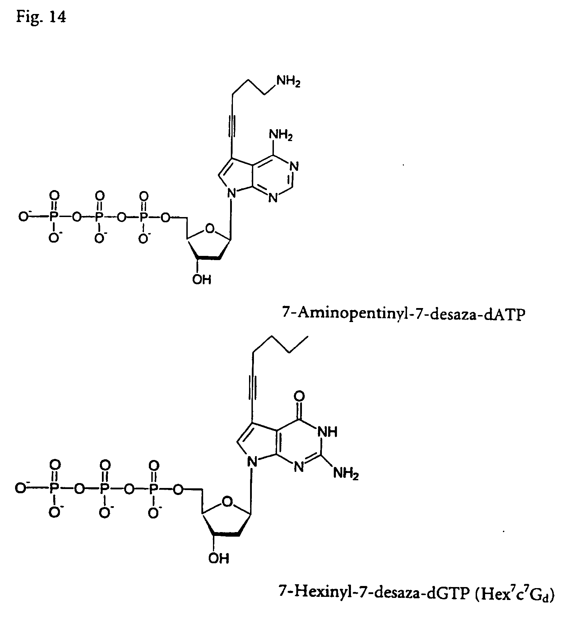

For structure formula, spacer compounds and molar masses see Figures 10-14 or RMB biochemicals catalogue for detailed information.

These modified dNTP's completely replaced the natural by occurring dNTP's in the labeling reactions completely.

The polymerase reactions are carried out in PCR tubes (200 µl) in a total volume of 10 µl, PCR reactions in a volume of 50 to 100 µl. Template and primer are annealed at 20 °C to 35 °C for 10 min. DNA polymerase concentrations were 0.1, 0.5 units or 1 units as indicated per reaction for *Taq, Chy* Klenow enzyme and *Vent* and *Vent exo*⁻ polymerase, 0.1 or 1 unit *Tgo exo*⁻ polymerase, (one unit is the incorporation of 10 nmol total deoxynucleoside triphosphates in acid precipitable DNA within 30 min. at the temperature used for each polymerase), for labeling using PCR the optimal enzyme concentration may be increased e.g. 1 to 4 units of enzyme may produce better incorporation rates. The working concentration of template/primer can be 0.5 to 1 pmol, as indicated. Template concentrations may vary between 0.1 to 0.75 µg and primer between 50 to 600 nM. The concentrations of the modified nucleotides may vary from 5 µM to 50 µM, unmodified dNTPs between 50 µM and 300 µM. As buffers Tris, bicine, tricine can be used in concentrations of 10 mM to 50 mM, the pH may be adjusted with HCl or acetic acid to values between 8.5 and 9.2. KCI, 0 to 100 mM, and/or (NH)₂SO₄, 0 to 20 mM, and MgCl, 1 to 4 mM or MnCl₂, 0.5 to 1.5 mM, Tween, 0.05 % to 2 %, and optionally DMSO, 1 to 5 %, or BSA, 100 µg/ml are used in the reaction mixture. The reaction mixtures may be incubated for 10 min. to 60 min. at a temperature optimal for the polymerase used e.g. *Chy* Klenow polymerase is used in the temperature range between 55°C to 72°C. For PCR labeling the hybridization temperature may vary with the sequence of the primer used. The elongation temperature and the number of cycles may vary with the thermostable polymerase and the template chosen.

The preferred primer extension-reactions are described in the following:

Template/primer strands were annealed at room temperature for 10 min. The polymerase reactions were carried out in PCR tubes (200µl) in a total volume of 10 µl. Each reaction contained 1 x polymerase buffer. DNA polymerase concentrations were 0.1, 0.5 units or 1 unit where indicated per reaction for *Taq, Chy* Klenow enzyme and *Vent* and *Vent* exo⁻ polymerase, 0,1 or 1 unit *Tgo* exo⁻ polymerase, (one unit is the incorporation of 10 nmol total deoxynucleotide triphosphates in acid precipitable DNA within 30 min at the temperature used for each polymerase). The working concentrations of template/primer were 0,5 to 1 pmol, as indicated. The concentrations of the modified nucleotides varied from 5 µM to 50 µM. Regular dNTP's were 12.5 µM each. The reactions were usually incubated with the polymerases under the following conditions: *Taq*: 0.1 u, 72°C, 30 min.; *Chy* Klenow enzyme: 0.1 u, 72°C, 30 min.; *Pwo, Vent* exo⁻ DNA-polymerase, 0.1 u, 72°C, 30 min.

The polymerisation reactions were terminated by the addition of 10 µl formamide stopping solution (98% deionized formamide, 10 mM EDTA, 0.01% bromphenolblue) and the DNA was denatured by heating for 10 min at 95°C. Aliquots of 3 µl were loaded onto 12,5 % denaturing acrylamide/urea sequencing-gels and electrophoresed at 2000-2500 Volts until the bromphenolblue dye reached the anode buffer tank.

Preferred conditions for digoxigenin detection:

After electrophoretic separation of the polymerase reaction products, DNA was transferred onto positively charged nylon membranes (RMB) by contact blotting for 30-60 min and crosslinked by irradiation (254 nm, 10-15 min). Then the membrane was blocked for 30 min in 0.1 M maleic acid, 0.15 M NaCl, pH 7.5 (buffer 1), containing 1% (w/v) blocking reagent (casein) and reacted with a 1: 10000 dilution of anti-digoxigenin-AP Fab fragments (RMB) for 60 min. Unbound antibody was removed by several washes with excess buffer 1 containing 0.3% tween 20. Then the membrane is transferred into buffer 2 (0.1 M Tris/HCl, pH 9.5, 0.1 M NaCl), washed again and finally incubated with CDP Star™ (1:1000 dilution in buffer 2) for 10-15 min. Excess fluid was carefully removed with a Whatman 3MM Chr paper. Then the blot was sealed between two sheets of transparencies and exposed to Lumi Film (RMB) or Lumi Imager™ (RMB) for 10 min. to 20 min.

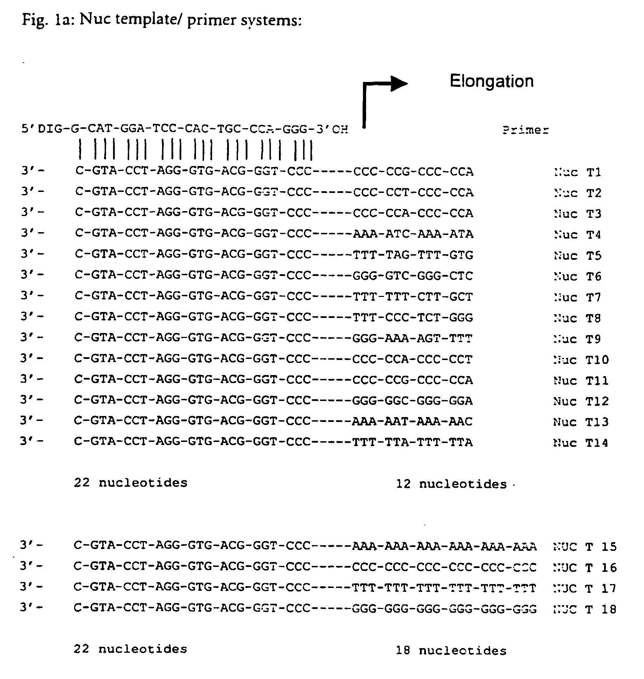

In order to determine the precise incorporation of the modified deoxynucleosidetriphosphates according to the instruction of the target DNA sequence well defined template/primer systems were used. This system enables to follow the elongation of a 5' digoxygenin labelled (22-mer) primer with different DNA polymerases in the presence of the modified derivatives. It was examined, if it is possible to incorporate several modified nucleotides one after another using a template with T-tracts (Nuc T15) or C-tracts (Nuc T16) and also further elongate with natural dNTPs (Nuc T-Templates, see fig.1a).

The template primer system cass 1-10 was used for incorporation studies where all four naturally occurring dNTP's were replaced by modified analogues. This template primer system consists of 10 template sequences, with building blocks of four bases, each represented once per unit, in that way, that cass 1 consists of one block, cass 2 of two and so forth. This system allows to follow the step by step incorporation off different derivatives (for example with cass 1) and to calibrate the reaction products up to 40 incorporation steps. The template sequences are permutated.

Therefore, the present invention also provides methods for testing modified deoxyribonucleotide triphosphates and polymerases with respect to their utility for high density labeling.

The method according to the present invention can be performed on solid or in liquid phase. The method according to the invention can be used for DNA synthesis and simultaneous DNA labeling by PCR.

The modified nucleotides incorporated into the newly synthesized nucleic acid are detectable by nonradioactive methods like excitation and detection of fluorescence, detection by an immunological reaction, detection by specific interaction (e.g. Streptavidin/Biotin) or by a chemically introduced label. It is preferred that the four modified bases carry different labels so that they can be distinguished one from another, e.g. dATP carries a modification different from the modification carried by dGTP, dCTP or dTTP.

Further, the inventive method can be used for DNA sequencing, especially single molecule sequencing. In one embodiment the invention can be used for single molecule DNA sequencing in submicrometer channels. This new method is based upon detection and identification of single fluorescently labeled mononucleotide molecules degraded from DNA-strands in a cone shaped microcappillary.

In case of single molecule sequencing of a high-density-labeled DNA the detection of the modified mononucleotide takes place after the degradation step that means after the modified mononucleotide was cleaved from the high-density-labeled DNA.

The method according to this invention can also be used for highly sensitive detection or quantification of one or several specific nucleic acid sequences.

Another use of the inventive method is the detection of specific nucleic acid sequences *in situ,* like PRINS or FISH.

With PRINS (Primed In Sit Labeling) an unlabeled synthetic oligonucleotide is annealed to the target sequence in denatured chromosomes in metaphase spreads or interphase nuclei on microscopic slides. The hybridized oligonucleotide serves as a primer which can be elongated with a thermostable DNA polymerase in situ incorporating labeled nucleotides in a high density into the newly synthesized DNA. Depending on the label(s) the newly synthesized DNA may be detected by various methods, e.g. indirect detection by fluorochrome conjugated anti-DIG antibody (if DIG-dUTP was incorporated), or direct detection with the fluorescence microscope (to detect e.g. incorporated Rhodamine-dUTP).

In the Fluorescence In Situ Hybridization (FISH) techniques fluorescently labeled DNA probes are used, which can be created by the inventive method. FISH can be used e.g. to detect certain sequences in whole chromosomes, to detect mRNA species or to detect viral RNA. A higher density of label which can be achieved by the method of this invention allows for the use of shorter probes which can diffuse into the dense matrix of cells or chromosomes more easily without loosing sensitivity. The acceptance of a whole spectrum of labeled nucleotides by the inventive method gives more flexibility in choosing the probe sequence and multiple labeling. Furthermore, it increases the sensitivity and the probability in detection of rare mRNA species.

Legend of Figures:
- Fig. 1a:: Nuc template/primer systems.
- Fig. 1b:: Cassettes 1-10 template/primer systems.
- Fig. 2:: Incorporation of Hexinyl-7-deaza-dATP and Hexinyl-7-deaza-dGTP.

Lanes 1-6: Control reactions with 0,1 unit *Taq*-polymerase per reaction. Lane1: Primer and buffer only; Lane 2: Template 17, complete reaction mix, all 4 dNTP's; Lane 3: as in lane 2, but dATP only; Lane 4: Template 16, complete reaction mix, dGTP only; Lane 5: as in lane 4, but with all 4 dNTP's; Lane 6: complete reaction mix, without template; Lanes 7-8: Template 17, 0,1unit polymerase, with 5 and 50 µM Hexinyl-7 deaza-dATP respectively; Lanes 9-10: Template 16, 0,1 unit polymerase, with 5 and 50 µM Hexinyl-7 deaza-dGTP respectively; Lanes 11-14: As lanes 7-10, but 1unit polymerase; Lanes 15-17: Template 8, 0,1 unit polymerase, 5 µM derivatisized Nucleotides Hexinyl-7 deaza-dATP (lane 15), Hexinyl-7 deaza-dATP and Hexinyl-7 deaza-dGTP (lane 16), Hexinyl-7 deaza-dATP, Hexinyl-7 deaza-dGTP and Rosamin-dCTP (lane17); Lanes 18-20: As lanes 15-17, but 50 µM dNTP's; Lanes 21-26: as lanes 15-20, but 1 unit polymerase; Lanes 27-34: template 3; Lane 27: Hexinyl-7 deaza-dGTP (50 µM), 0,1 unit polymerase; Lane 28: Hexinyl-7 deaza-dGTP and Biotin dUTP (50 µM each), 0,1 unit polymerase; Lanes 29-30: As lanes 27-28, but 1 unit polymerase; Lanes 31-32: Hexinyl-7 deaza-dGTP and Fluorescein-12-dUTP (50 µM each), 0,1 and 1 unit polymerase respectively; Lanes 33-34: Hexinyl-7 deaza-dGTP and AMCA-6-dUTP (50µM each), 0,1 and 1 unit polymerase respectively.

Compound: A: Hexinyl-7-deaza-dATP; B: Hexinyl-7-deaza-dGTP; C: Rosamin-dCTP D: Biotin-16-dUTP E: Fluorescein-12-dUTP; F: AMCA-6-dUTP.

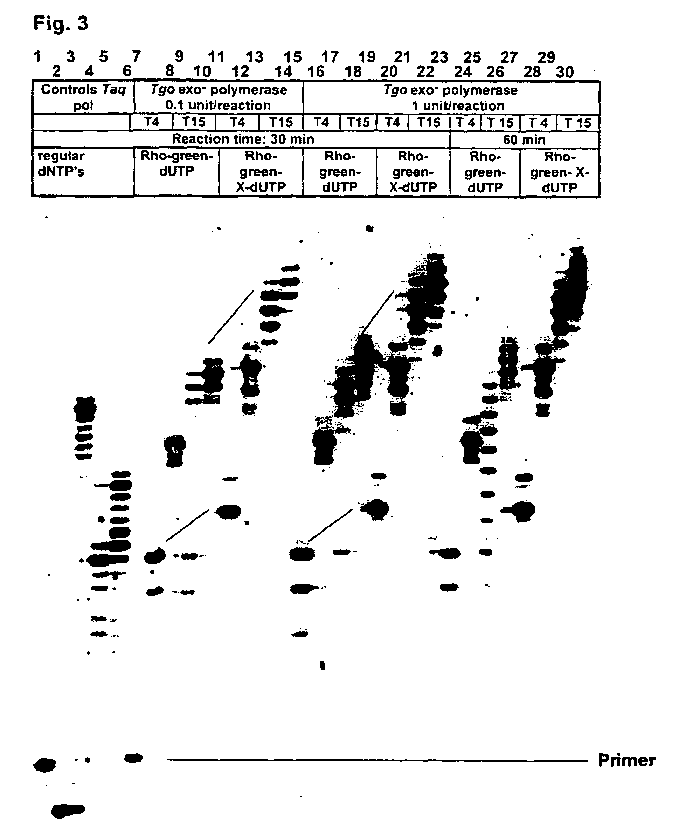

### Fig. 3: Rhodamin-green-dUTP incorporation by Tgo exo⁻ polymerase.

Multiple incorporations of Rho-green and Rho-green-X-dUTP in adjacent positions and comparison of linker length on substrate activity.
Lanes 1-6: Control reactions. Lane 1: Dig-labeled Primer (22-mer) and buffer only; lane 2: Dig-labeled Primer (20-mer) and buffer only; lane 3: Template / primer (28 incorporations possible), Taq-polymerase and 4 regular dNTP's; lane 4: Template / primer (12 insertions possible), Taq-polymerase and 4 regular dNTP's; lane 5: Template / primer (18-times insertion of dTTP or dUTP possible), Taq-polymerase and 4 regular dNTP's; lane 6: as lane 1; lanes 7-14: Reactions with 0.1 unit Tgo exo⁻ polymerase; lanes 7-10: Rhodamin green-dUTP; lanes 11-14: Rhodamin green-X-dUTP; lane 7: Template Nuc T4, 5µM Rhodamin green-dUTP; lane 8: Template Nuc T4, 50µM Rhodamin green-dUTP, dATP, dGTP; lane 9: Template Nuc T15, 5µM Rhodamin green-dUTP; lane 10: Template Nuc T15, 50µM Rhodamin green-dUTP; lane11: Template Nuc T4, 5µM Rhodamin green-X-dUTP; lane 12: Template Nuc T4, 50µM Rhodamin green-X-dUTP, dATP, dGTP; lane 13: Template Nuc T15, 5µM Rhodamin green-X-dUTP; lane 14: Template Nuc T15, 50µM Rhodamin green-X-dUTP; lanes 15-22: as lanes 7-14, but 1u Polymerase; lanes 23-30: as lanes 15-22, but 60 min reaction time.

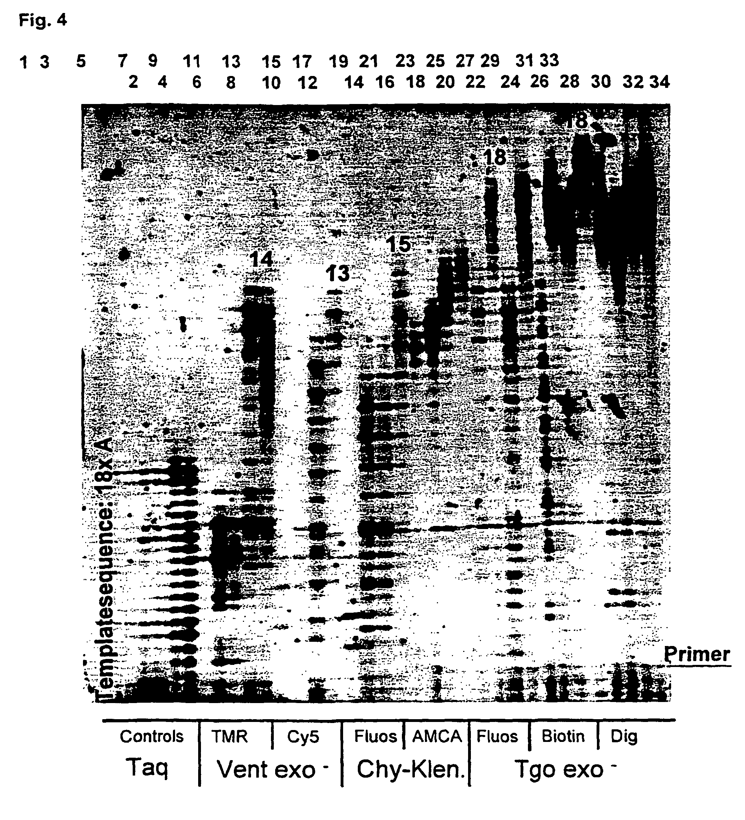

### Fig. 4: Incorporation of different U-derivatives by different polymerases.

Different U-derivatives are efficiently incorporated by different polymerases in neighbouring positions on the template NucT15.

Lanes1-6: Control reactions. Lane 1: Primer and buffer only. Lanes 2-6 contained 0,1 unit Taq polymerase per reaction. Lane 2: 13,5 µM dATP; Lane3: 12,5 µM dGTP; Lane 4 and 5 are identical with 12,5 µM of each natural dNTP; Lane 6: As lanes 4 or 5, without template; All other labelling reactions contained only the derivatisized nucleoside triphosphate indicated. Lanes 7-10: Incorporation of Tetramethylrhodamine with *Vent* exo⁻ polymerase. Lane 7: 5 µM TMR-dUTP and 0,1 unit polymerase; Lane 8: 50 µM TMR-dUTP and 0.1 unit polymerase; Lanes 9,10: As lanes 7,8, but with 1 unit polymerase. Lanes 11-14: As lanes 7-10, but with Cy5-dUTP; Lanes15-18: Incorporation of Fluorescein-dUTP with *Chy*-polymerase Klenow- fragment. Lanes 15, 16: 0,1 unit polymerase with 5 µM and 50 µM Fluorescein-dUTP respectively; Lanes 17,18: As lanes 15, 16 but with 1 unit polymerase; Lanes 19-22: As lanes 15-19 with AMCA-dUTP as substrate; Lanes 23-34: Incorporation with *Tgo* exo⁻ polymerase; Lanes 23-26: As lanes 15-19 with Fluorescein-dUTP as substrate; Lanes 27-30: As lanes 15-17 with Biotin-dUTP as Substrate; Lanes 31-34: As lanes 15-17 with Digoxigenin-dUTP as subtrate.

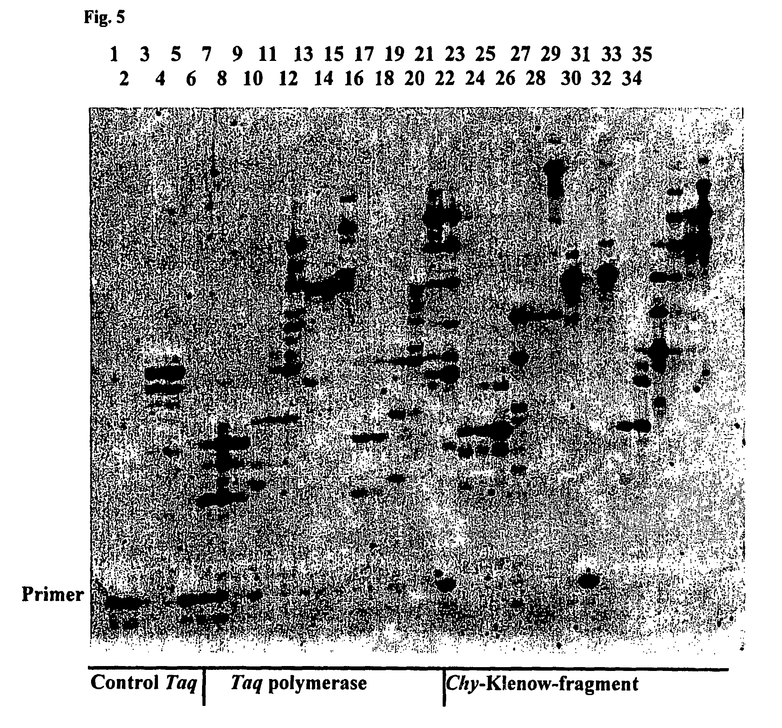

### Fig. 5: Incorporation of Cy5-dUTP with different spacer lengths by Type A polymerases.

Comparison of incorporation efficiency of Cy5-dUTP with different linker lengths by A-type polymerases.

Lanes 1-5 : Control reactions. Lanes 1 and 2: no polymerase; Lane 1: additionally no template; Lane 3 and 4: Complete reaction mix with 4 dNTP's and template NucT4; Lane 5: Complete reaction mix, no template; Lanes 6-20: *Taq* polymerase; Lanes 6-14: Template NucT4; Lanes 15-20: Template T15; Lanes 6-8: Cy5-dUTP with 17- atom spacer; Lane 6: 5 µM Cy5-dUTP; Lane 7: 50 µM Cy5-dUTP; Lane 8: 50 µM Cy5-dUTP plus 12,5 µM dATP and dGTP; Lanes 9-11: As lanes 6-8, but with Cy5-dUTP with 24-atom spacer; Lanes 12-14: As lanes 6-8, but with Cy5-dUTP with 38-atom spacer; Lanes 15,16: Template NucT15, with 5 and 50 µM 17-atom spacer Cy5 dUTP respectively; Lanes 17,18: As lanes 15,16, with 5 and 50 µM 24-atom spacer Cy5 dUTP respectively; Lanes 19, 20: As lanes 15,16, with 5 and 50 µM 38-atom spacer Cy5 dUTP respectively; Lanes 21-34: As lanes 6-20, but with *Chy*-Klenow polymerase instead of *Taq* polymerase.

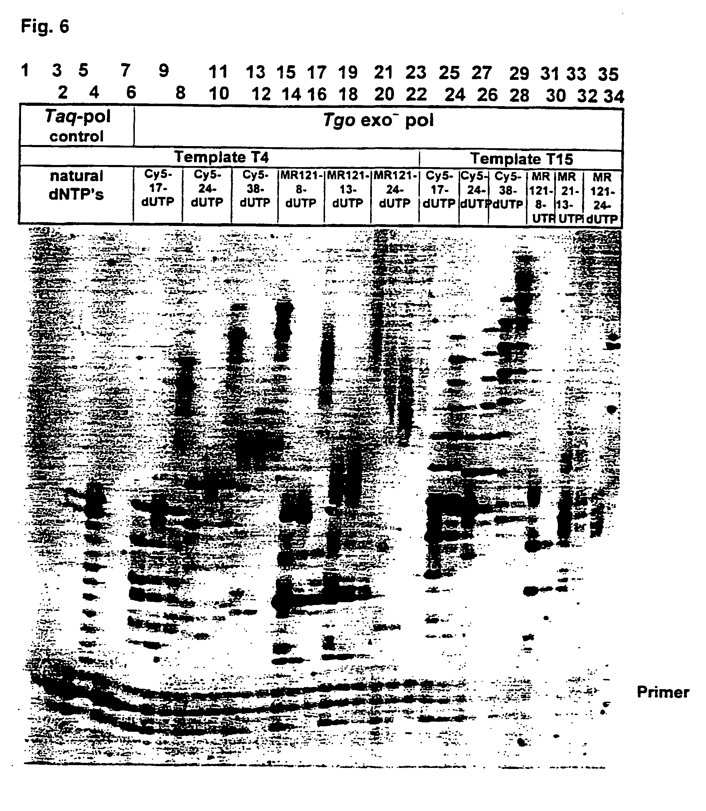

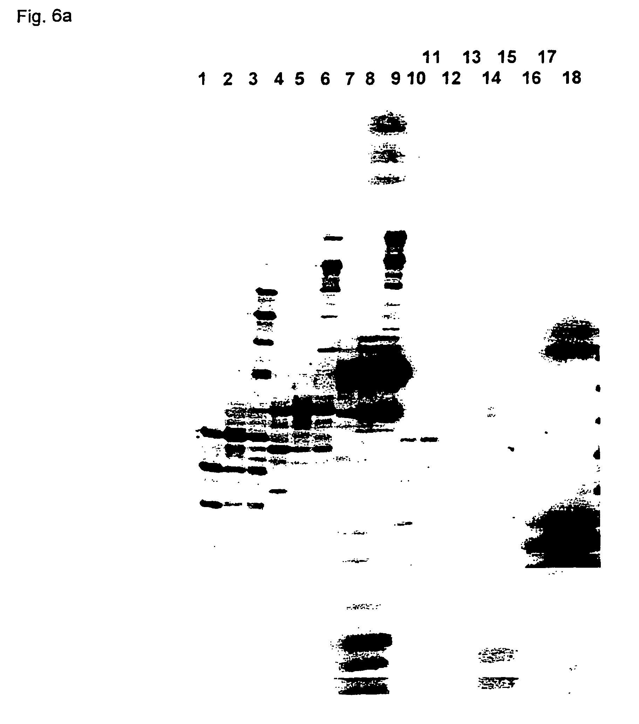

### Fig. 6: Incorporation of Cy5-dUTP and MR121-dUTP with different spacer lengths by an exo⁻ Type B polymerase

Comparison of incorporation efficiency of Cy5-dUTP and MR121-dUTP with different linker lengths by B-type polymerases.

Lanes 1-5: Control reactions with 0,1 unit *Taq*-pol. Lane 1: Primer alone; Lane 2:Template/primer, no nucleotides; Lane3: Complete reaction mix; Lane 4: As lane 3, without pol; Lane 5: As lane 3, without pol; Lanes 6-8: Cy5-dUTP with 17 atom spacer; Lane 6: 5 µM Cy5-dUTP; Lane 6: 50 µM Cy5-dUTP; Lane 8: 50 µM Cy5-dUTP plus dATP and dGTP; Lanes 9-11: As lanes 6-8, but Cy5-dUTP with 24 atom spacer; Lanes 12-14: As lanes 6-8, but Cy5-dUTP with 38 atom spacer; Lanes 15-23: As lanes 6-14, but MR121 dUTP with 8, 13, 24 atoms linker length instead of Cy5-dUTP; Lanes 24-35: Template NucT15; Lane 24: 5 µM Cy5-dUTP with 17 atom spacer; Lane 25: 50µM Cy5-dUTP with 17 atom spacer; Lanes 26-27: As lanes 24-25, but Cy5-dUTP with 24 atom spacer; Lanes 28-29: As lanes 24-25, but Cy5-dUTP with 38 atom spacer; Lanes 30-35: As lanes 24-29, but MR121-dUTP with 8, 13, 24 atoms linker length instead of Cy5-dUTP.

### Fig. 6a: Fluorescence scan of the blot of fig. 6 prior to chemiluminescence detection.

The scan comprises lanes 6-23 of fig. 6 and was analyzed prior to immunological detection of digoxigenin labeled primer, as it was shown, that blocking procedure interferes with fluorescence analysis. Lanes 1-9: Incorporation of Cy5-dUTP in Template NucT4 by *Tgo* exo⁻ polymerase. Lanes 1-3: 17-atom spacer; lanes 4-6: 24-atom spacer; lanes: 7-9: 38-atom spacer. Incorporation of MR121-dUTP in template T4 by *Tgo* exo⁻ polymerase. Lanes 10-12: MR121-8-dUTP; lanes 13-15: MR 121-13-dUTP; lanes 16-18 MR 121-24-dUTP.

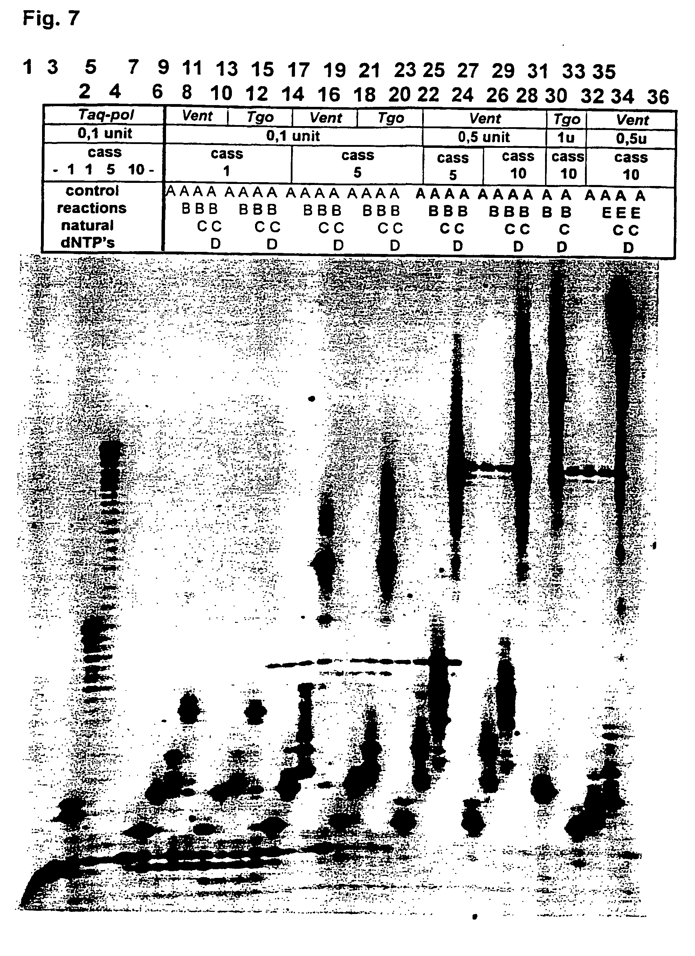

### Fig.7: Full replacement of all four Desoxynucleoside-triphosphates by derivatisized dNTP's

Polymerases used were *Vent exo*⁻ and *Tgo exo*⁻ for labeling reactions (lanes 7-36) and *Taq* pol for control reactions (lanes 1-6). Derivatives used were: A: Hexinyl-7-deaza dGTP (5µM and 50µM, as indicated) instead of dGTP. B: Rosamin-dCTP (5µM and 50µM, as indicated) instead of dCTP. C: Hexinyl-7-deaza dATP (5µM and 50µM, as indicated) instead of dATP. D. Biotin-16-dUTP (5µM and 50µM, as indicated) instead of dTTP. E: dCTP, no label, in reactions 34-36 as a control.

### Fig. 8: Synthesis of a DNA strand consisting of four differently derivatisized desoxy-nucleotide triphosphates.

Documentation of synthesis of a DNA of 40 nucleotides target sequence with all dNTP's replaced by derivatized nucleotides.

Lanes 1-4: Control reactions; Lane 1: Primer only; Lanes 2, 3: complete reaction mix with 0,1 unit Taq polymerase and 12,5 µM each of natural dNTP's; Lane 5: As lane 1; Lanes 5-14: Labeling reactions with *Tgo* exo⁻ polymerase (0,1 unit per reaction) cass I in lane 5, cass 2 in lane 6, cass 3 in lane 7, cass 4 in lane 8, cass 5 in lane 9, cass 6 in lane 10, cass7 in lane 11, cass 8 in lane 12, cass 9 in lane 13 and cass 10 in lane 14 as template; Derivatives (each 50µM) were: Amino-pentinyl-7-deaza-dATP instead of dATP, Hexinyl-7-deaza-dGTP instead of dGTP, Digoxigenin-dCTP instead of dCTP and Biotin-16-dUTP instead of dTTP; Reaction time 30 min.; Lanes 15-24: same as lanes 5-14 but 60 min. reaction time.

### Fig.8a: In of migration distance of separated products of Fig.8

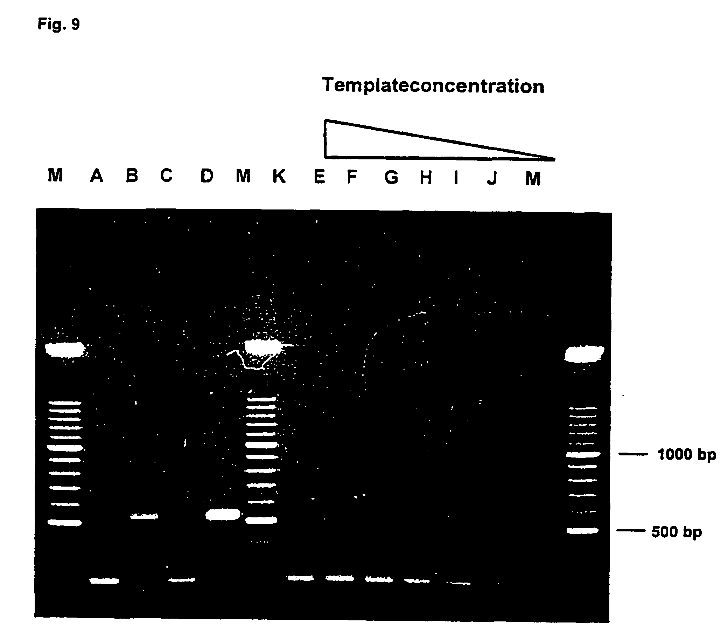

### Fig. 9: Generation of a 264 bp-fragment by PCR with complete substitution of dTTP by Biotin-16-dUTP.

Incorporation of modified nucleotides in PCR (complete substitution).

Lanes marked with M are Marker lanes. Lane A: Control reaction with natural dNTP's; Lane B: 264 bp fragment with dTTP completely replaced by Biotin-16-dUTP; Lane C: 264 bp product with natural dNTP's using excised and purified product of lane B as template (reversion); Lane D: As lane C, but dTTP completely replaced by Biotin-16-dUTP; Lane K: As lane A (control); Lanes E-J: As lane C, but with serial dilution (10-fold) of added template (Template titration).

### Fig. 10-14: Formulae of derivatives used.

### Fig. 15: List of nucleoside derivatives used.

### Example 1:

### Synthesis of 7-deazapurine-2'deoxynucleoside 5-triphoshate derivatives:

### General:

Thin layer chromatography (TLC): TLC aluminium sheets silica gel 60 F₂₅₄ (0.2 mm, Merck, Germany). Reversed phase HPLC was carried out on a 4x250 mm RP-18 (10µm) LiChrosorb column (*Merck*) with a *Merck-Hitachi* HPLC pump (model 655 A-12) connected with a variable wavelength monitor (model 655-A), a controller (model L-5000), and an integrator (model D-2000). ³¹P NMR spectroscopy was carried out on an AC-250 Bruker NMR spectrometer. 12.5% denaturing Polyacrylamide gel electrophoresis (Sequagel/ National Diagnostics) was carried out on a DS91 Sequencer (Biometra, Germany) connected to a PS 2500 DC Power Supply (Hoefer Scientific Instruments, USA) and a Lauda MGW thermostat (Lauda, Germany) or with a Pharmacia LKB Macrophor sequencing gel unit with a ECPS 3000/150 power supply (Pharmacia/LKB) connected with a Haake D1 thermostat.

Preparative denaturing gel electrophoresis for primer purification was carried out with 15% acrylamide/ urea vertical slab gels (15cm x 20cm x 0.2cm; Sequagel/ National Diagnostics) and electrophoresed at 150-200 volts (Consort microcomputer electrophoresis power supply) until the bromphenolblue dye reaced the anode buffer reservoir ³¹P NMR spectroscopy was carried out on a AC-250 Bruker NMR spectrometer.

### Synthesis of the 5'-Monophosphates:

1.0 mmol of the modified nucleosides was dissolved in trimethylphosphate (5 ml) under argon. After cooling down in an ice bath freshly destilled POCl₃ (180 µl) was added and the reaction kept at 4°C for several hours. (TLC control after hydrolysation of a small portion with TEAB-buffer in i-propanol/H₂O/NH₃ (3/1/1) or (7/1/1)). When the reaction was complete, the solution was added dropwise to TEAB-buffer (200 ml) while cooling. After evaporation the residue was dissolved in H₂O and applied to a DE 52 Cellulose (Whatman) column (20 x 3 cm)). After washing with 11H₂O purification was accomplished using a gradient of 11H₂O and 111M TEAB-buffer. The 5'-monophosphate eluted from the column at a TEAB concentration of 0.3 - 0.4M.

The product was lyophilised and characterised by³¹P-NMR.

### Synthesis of 5'-Triphosphates:

The 5'-monophosphate was dissolved or suspended in DMF p.a. (11 ml) and treated under argon with a solution of 1,1 1'-carbonyldiimidazole (0.5 mmol, 80 mg) in DMF (1ml). The mixture was shaken in a well sealed vessel for 30 min and then kept in an argon floated exicator at rt. over night. Methanol (33 µl) was added under argon and after 30 min at rt. mono-(tri-n-butylammonium)pyrophosphate (0.5 mmol, 0.1816 g) in DMF p.a. (5 ml) was added while stirring. The reaction mixture was kept in an argon floated exicator over night leading to a precipitation. The supernatant was separated and the precipitate washed 4 times with 1 ml DMF each and centrifuged. The combined washings were evaporated, dissolved in H₂O and applied to a DE 52Cellulose column (20 x 3 cm). After washing the column with 11H₂O purification was accomplished using a gradient of 11 H₂O and 111M TEAB-buffer. The desired product eluted from the column at a TEAB-concentration of 0.2-0.4 M. The 5'-triphosphate was dried by lyophilisation and characterised by ³¹P-NMR.

**Deprotection of the Amino group of the Trifluoroacetyl protected compounds:**

The 7-trifluoroacetylaminopentinyl-7-deazapurine-2'deoxynucleoside 5-triphosphate was dissolved in H₂O (12.5 ml) and treated with conc. ammonia (12.5 ml) under stirring for 3.5 h. The solution was stirred under aspirator vacuum for 2 h to remove the ammonia and lyophilised. The residue was dissolved in 0.1 M TEAB (10 ml, pH 7.8) and applied to a DE 52 Cellulose column (20 x 3 cm). The column was eluted with a linear gradient of 0.1 M TEAB (11) to 1.0 M (11). The fraction of the main zone were evaporated, co-evaporated with ethanol and lyophilised.

**Table 1:**

| Yield and ³¹P NMR data of the 7-substituted-7-deaza-2'-deoxyadensine and guanosine 5'-Mono- and Triphosphates | | |
|---|---|---|
| Compound | Yield [%] | ³¹P NMR ppm (J [Hz]) |
| Hex⁷c⁷A_{d} 5'-MP | 61 | 3.99 |
| Hex⁷c⁷A_{d} 5'-TP | 10 | -21.30 (t, 19.97); -10.26 (d, 19.31); -6.28 (d, 20.66) |
| NH₂Pen⁷c⁷A_{d} 5'-MP^{a)} | 20 | 4.09 |
| NH₂Pen⁷c⁷A_{d} 5'-TP | 35^{b)} | -21.25 (t, 20.08); -10.30 (d, 19.88); -6.16 (d, 19.33) |
| Hexc⁷G_{d} 5'-MP | 20 | 3.65 |
| Hex⁷c⁷G_{d} 5'-TP | 87 | -21.34 (t, 19.23), -10.25 (d, 19.41), -6.40 (d, 20.51) |
| NH₂Pen⁷c⁷G_{d} 5'-MP^{a} | 10 | 4.11 |
| NH₂Pen⁷c⁷G_{d} 5'-TP | 20^{b} | -21.00 (t, 19.24), -10.20 (d, 19.21), -5.65 (d, 19.28) |

| | | |
|---|---|---|
| a) trifluoroacetyl protected; | | |
| b) after deprotection of the amino group | | |

### Example 2:

### Synthesis and purification of template oligonucleotides:

The solid phase synthesis of oligonucleotides was carried out on a 1 µmol scale on an automated DNA synthesiser (Applied Biosystems, ABI 392-08) using phosphoramidite chemistry. The phosphoramidites of dG, dA, dC, and T and the CPG columns were purchased from PerSeptive Biosystems GmbH (Germany). Aminolink II (Applied Biosystems, 100 mM solution in CH₃CN)) was used for further 5'-labelling of the primer. All oligonucleotides were synthesised in a tritylon synthesis followed by cleavage from the support and deprotection with ammonia (25 %, 60°C, 18 h).

The purification of template strands was performed using a OPC (Oligonucleotide Purification Cartridge) column (ABI Masterpiece, Applied Biosystems, Germany) following the purification protocol given there. The oligonucleotides were further purified by polyacrylamide gel electrophoresis (PAGE) on a 15 % gel in the presence of 7 M urea and visualised by UV shadowing with PSC 60F₂₅₄₊₃₆₆ (Merck 5637 thin layer chromatography plates); the target length product bands were excised and eluted from the gel in 0.5 M ammonium acetate, 1 mM EDTA, 0.1% SDS). The resulting oligonucleotides were concentrated and desalted using an OPC cartridge or ethanol precipitated and diluted in an appropriate volume of water. The latter step turned out to be of crucial importance, as shortened template oligonucleotides (due to coupling errors) could fake synthesis stops, and thus preventing appropriate evaluation of data presented here. The purified oligonucleotides were lyophilised on a Speed-Vac evaporator to yield colourless solids which were dissolved in 100µl of H₂O and stored frozen at -18°.

### Example 3:

### Labeling and purification of the primer:

After deprotection of the oligonucleotide the ammonia was removed by evaporation and desalted by ethanol precipitation. 20 A₂₆₀ units of the oligonucleotide were dissolved in 200 µl of 100 mM sodium borate (pH 8.5) and treated with a freshly prepared solution of 350 mg DIG-NHS in 200 µl ethanol. The reaction was kept in a shaker overnight at room temperature, concentrated and the residual fluid dissolved in 1 ml H₂O. After filtration through a 0.45 µm filter the solution was applied to reversed phase HPLC using the following gradient: 20 min 100% 0.1 M Et₃NHOAc (pH 7.0) (A), 20-50 min 0-40 % CH₃CN in A. The unlabeled oligonucleotide elutes at a CH₃CN-concentration of ca 20%, the DIG-labelled oligonucleotide at ca 30% CH₃CN. The DIG-labelled primer is desalted and gel purified as described above.

### Example 4:

### Incorporation of the 7-substituted 7-deaza-2'-deoxyadenosine and -guanosine derivatives:

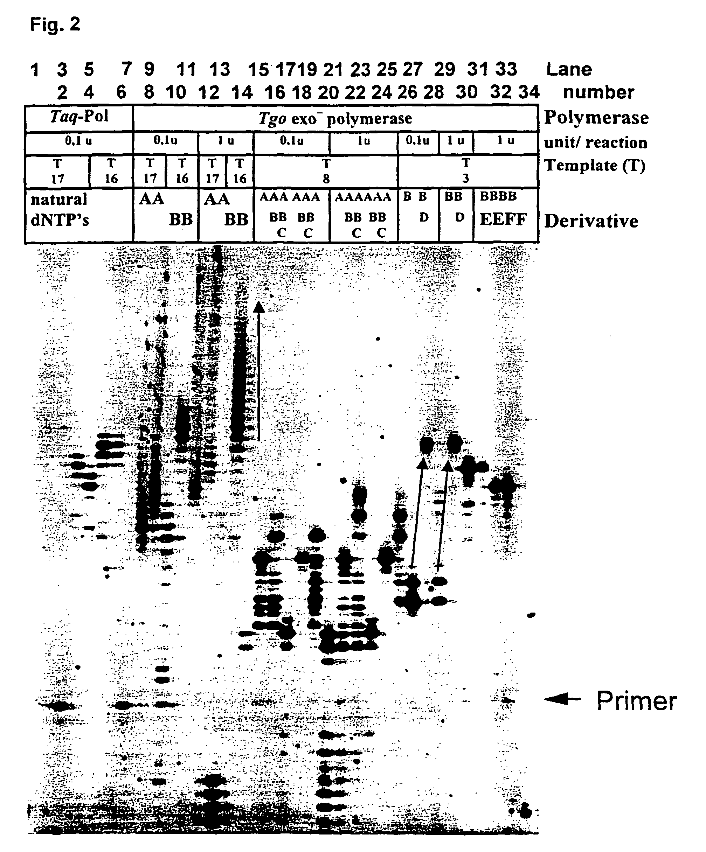

In fig 2 the incorporation of 7-substituted 7-deaza-2'-deoxyadenosine and -guanosine derivatives and elongation with other derivatized nucleotides by *Tgo* exo⁻ polymerase was analyzed. For this purpose template oligos NucT17, T16, T8 and T3 were used. This allowed to monitor the effective incorporation of the respective triphosphates in up to 18 adjacent positions and further elongation with other modified deoxynuclesidetriphosphates.

According to the base pairing rules the 2'-deoxyadenosine derivative (50 µM) was incorporated into 15 adjacent positions using template NucT17 (lanes 7-8) and the 2'-deoxyguanosine derivative could be polymerized in up to 21 subsequent positions using NucT16 template (fig. 2, lanes 9-10). In lanes 15-34 the further elongation of the 7-deaza-compounds with other derivatized deoxynuceoside-triphosphates was analyzed. Nuc T 8 was chosen to demonstrate the successful addition of Hex⁷c⁷G_{d} on a track consisting of three consecutive Hex⁷c⁷A_{d} and further elongation with Rosamin-dCTP. Lanes 15, 18, 21 and 24 show the reaction products with Hex⁷c⁷A_{d} being the exclusive deoxynucleotide-triphosphate present in the reaction mixture. Depending on the polymerase amount and nucleotide concentration the polymerase adds the dNTP's as expected, but the products exceeded the template instructed length of three Hex⁷c⁷A_{d}'s (by one and to a minor extent two polymerisation steps) indicating, that the polymerase incorporates up to two mis-paired Hex⁷c⁷A_{d} opposite to C in the template, wich is a commonly observable phenomenon in a extremely biased nucleoside triphosphate pool.

Lanes 16, 19, 22 and 25 show the disappearing of the expected and non expected bands of the previous reactions (see above) and accumulation of higher molecular weight material, when the reactions contained additional Hex⁷c⁷G_{d}. An estimation, counting the intermediate stops of lanes 16 and 22 revealed, that according to the instruction of the template 9 incorporations were detectable. In reactions containing 50 µM of the respective derivative (lanes 19 and 25) almost no stops were observed and a homogenous band is shown.

Further addition of Rosamin-dCTP to Hex⁷c⁷A_{d} and Hex⁷c⁷G_{d} in reactions 17, 20, 23, 26 showed in all reactions again a further elongation of the final product of the previous reactions. In the case of lane 23 (1u polymerase) the expected three consecutive incorporations of Rosamin-dCTP could be detected. This means, that a DNA stretch has been consecutively built up, consisting of 12 derivatized nucleotides.

Lanes 27-34 show similar experiments, using template NucT3. Here five (as instructed by the template) and six (one additional polymerisation step, due to biased nucleotide pool) consecutive incorporations of Hex⁷c⁷G_{d} are observed. When Biotin-16-dUTP is added to the reaction mixture (lanes 28 and 30) the stops disappear and a high molecular weight product is formed, exceeding the molecular mass of that of reaction 23 (nine Hex⁷c⁷A_{d}, Hex⁷c⁷A_{d} plus three Rosamin-dC's), indicating, that also the terminal base has been inserted (indicated by arrows). Lanes 31-34 show reactions with Biotin-16-dUTP replaced by derivatives with decreasing linker lengths (i.e. Fluorescein-12-dUTP, lanes 31,32 and AMCA-6-dUTP, lanes 33, 34). Here complete elongation can be deduced, no significant pausing or stop sites are detected.

At higher polymerase concentrations more than 30 insertions (lane 14) were observed. This might be due to a looping back mechanism of the elongated primer strand and rehybridisation at elevated temperatures. A control reaction with Taq polymerase and natural dNTP's revealed 22 insertions with NucT16 (lanes 4 and 5). Klenow fragment was able to synthesize up to 19 natural dNTP's under similar conditions with NucT15, but at 37°C (data not shown).

With this experimental setup it could be shown, that DNA-strands consisting of Hex⁷c⁷A_{d} and Hex⁷c⁷G_{d} could be readily elongated with other derivatized dNTP's yielding full length products.

### Example 5:

### Incorporation of Rhodamine green-dUTP and Rhodamine green-X-dUTP by Tgo exo⁻-polymerase.

In this experiment incorporation and elongation of two Rhodamin-green derivatives with natural deoxynucleosidetriphosphates were examined. The reaction was catalyzed by *Tgo* exo⁻-polymerase in the presence of 0,05% Triton X-100, which has been shown to enhance the incorporation efficiency in a separate experiment (data not shown). The two nucleotide-conjugates differed in their spacer compound. Rhodamin green-dUTP (Rhodamin-green-5(6)-carboxyamido-[5-(3-aminoallyl)-2'desoxy-uridin-5'-triphosphate])has a 5 atom spacer group and Rhodamin-X-dUTP (Rhodamin-green-5(6)-carboxyamido-ε-aminocaproyl-[5-(3-aminoallyl)-2'desoxy-uridin-5'-triphosphate]) a 12-atom spacer group between the desoxynudeoside compound and the fluorophore molecule.

Fig. 3 shows, that both compounds were effectively incorporated. The mass differences between these two are indicated by arrows between lanes 7, 11 and lanes 15, 19 respectively. It is shown, that with template NucT4 the polymerisation stops after 4 insertions (lanes 7, 11, 15, 19, 23, 27). Some minor false incorporations could be detected in position 5, where in the template sequence is a Thymidine residue (lanes 11, 19, 27). Beyond this misincorporation no further elongation is detectable. The major stop in position 4 can be overcome, when to the reaction mixture additionally dATP and dGTP is added. A higher molecular product is formed with both derivatives (lanes 8, 12, 16, 20, 24 and 28). But with this template there is still an ambiguity, wether a full lenghth product is formed (12 insertions, 9 of them being the derivative). The data here would favour the interpretation, that the polymerase stopped in position 11 and that the last derivative is added only very poorly in position 12. No major differences could be detected, whether 0,1 or 1 unit polymerase was used, or the reaction time was prolonged from 30 to 60 min. Only the amount of full-length-product increased, when longer incubation times and more polymerase were used.

In the case of template NucT15 (18 subsequent insertions possible) the derivative with the short linker arm was inserted in up to 15-16 neighbouring positions (compare lanes 25 and 26). Rhodamine- green -X-dUTP on the other hand was polymerized in up to 18 positions, with major products of 13-17 insertions (lane 30). The stops below are detectable only after overexposition of the film, indicating, that this nucleotide is a very good substrate for the polymerase and does not lead to abortive DNA synthesis. It is also assumed, that the linker length might have some influence on the substrate acceptance by the polymerase. In this case, one would favour, that the longer linker compound results in a better acceptance as a substrate. This is not only documented in the possibility of the polymerase to insert up to 18 nucleotides for Rhodaminegreen-X-dUTP instead of 16 for Rhodamine-green-dUTP, but also, when lanes 7,15 and 22 are compared with lanes 11, 19 and 27. In the case of Rhodamine-green-X-dUTP no stops are detectable up to the insertion of the fourth nucleotide, whereas stops after the incorporation of 2 and 3 nucleotides are detectable with Rhodamine-green-dUTP.

### Example 6:

### Incorporation of different U-derivatives by different poymerases.

In this example the ability of different types of polymerases was analyzed to incorporate different reporter group-tagged nucleotides. The polymerases used were two closely related B-type polymerases of different *Thermococcus* species (3'-5' exo minus variants) and a genetically shortened form (Klenow-fragment, lacking 5'-3'-nuclease activity) of an A-type polymerase of *Carboxydothermus* hydrogenoformans. The experiments carried out here, used a template consisting of 18 Adenine residues, starting from the 3'-primer terminus, enabling the polymerase to incorporate 18 of the variant nucleotides in neighbouring positions. Reactions were carried out with 5 µM and 50 µM modified nucleotides (final concentration) and 0,1 and 1 unit polymerase respectively. To the reaction buffer (50 mM Tris-HCL, pH = 8.5 at 20°C, 10 mM KCI, 15 mM (NH₄)₂SO₄, 7 mM MgSO₄ and 10 mM 2-Mercaptoethanol, used for each polymerase) 0,01% (vol/vol) Triton® X-100 was added to enhance incorporation.

Figure 4 shows, that with the derivatives TM-Rhodamine-dUTP, Cy5-10-dUTP, Fluorescein-12-dUTP and AMCA-6-dUTP at higher polymerase concentrations (1 unit/ reaction) longer products were synthesized than with 0,1 unit/ reaction (lanes 7,8; 11,12; 14,16; 19,20; compared with lanes 9,10; 13,14; 17,18 and 21,22 respectively). In the reactions with Fluorescein-12-dUTP, Biotin-16-dUTP or Dig-11-dUTP and *Tgo* exo⁻-polymerase also with lower polymerase concentrations (0,1 unit/ reaction) full length products were detectable (lanes 28 and 31).

In the case of AMCA-6-dUTP (lanes 19-22) and Dig-11-DUTP (lanes 31-34) the amount of virtually incorporated nucleotides could not be determined exactly. This is due to the lack of stops during synthesis under otherwise suboptimal reaction conditions (5 µM modified dNTP; 0,1 unit polymerase). This indicates a high substrate activity of these compounds and a superiour incorporation performance compared with other derivatives (e.g. Cy5-10-dUTP).

The migration distances between the non elongated primer and the different products formed, vary substantially between the respective derivatives and the controls with natural dNTP's (e.g. lanes 4, 5) This represents to a large extent the different molar masses of the derivatives (note, that AMCA-dUTP is a comparatively small molecule (mw.: 748,1 Da) compared with Dig-11-dUTP (mw.: 1090,7 Da), explaining the higher mobility of full-length products in denaturing gel electrophoresis). TM-Rhodamine-dUTP and Cy5-10-dUTP were inserted into 14 and 13 positions respectively by *Vent* exo⁻-polymerase, with major amounts of products between 10 and 13 incorporations for TM-Rhodamine (lanes 9, 10) and 11 and 12 incorporations for Cy5-10-dUTP (lane 14). Suboptimal reaction parameters (5µM dNTP, 0.1 unit Polymerase) were chosen to faciliate the interpretation of the banding pattern of reaction intermediates (calibration), in order to document, whether full length synthesis has occurred (see lane 27). Biotin-16-dUTP was incorporated into all possible positions by *Tgo* exo⁻-polymerase. Lane 27 shows synthesis stops at low polymerase and nucleotide concentrations from position 1-10 (11, hardly visible on original film), allowing the proper determination of 18 incorporated nucleotides in lane 30. The observed band splitting of each incorporation step is probable due to the existence of stereoisomers occuring in Biotin-16-dUTP preparation (Muehlegger, pers. communication).

In this example it was verified, that both, A- and B-type polymerases, are able to synthesize long stretches of derivatized nucleotides in adjacent positions, using high magnesium concentrations (7 mM) and detergent (0,01% vol/vol) Triton®-X-100. All products are shifted to higher molecular weights, compared with the products formed in the control reactions (lanes 1-6) with regular nucleotide-triphosphates, due to the higher molecular masses of the incorporated compounds. In this system, AMCA-, Biotin- and Dig-dUTP showed the best incorporation rates, followed by Fluoresceine-dUTP. If one compares the incorporation of the latter substance between the A-type (*Chy*-Klenow) and the B-type-polymerase (*Tgo* exo⁻), one can attribute a better incorporation performance to the engineered B-type polymerase (more insertions, less stops; see lanes 15-18 and 23-26). B-type polymerases (especially, when 3'-5' exonuclease function has been deleted) should be considered as the enzymes of choice in performing labeling reactions with derivatized dNTP's exclusively. 3'-5' exonuclease activity should be regarded as detrimental to labeling efficiency, due to degradation of primer and/ or template DNA. So deletion of 3'-5' exonuclease activity has to be regarded convenient, as wild type forms of these polymerases exhibit up to five times more 3'-5' exonuclease activity per unit DNA polymerase activity [1].

### Example 7:

### Incorporation of Cy-5-dUTP with different linker-arm lengths by A-type polymerases.

In this study the influence of the linker arm length on the incorporation by different polymerases (see also example 5) was investigated. As already pointed out in example 2, there might be some influence of the distance between nucleoside-compound and fluorophore/ detectable group on the enzymatic polymerisation of such a compound. For this study we used linker-arm lengths of the Cy-5-dUTP-derivatives of 17, 24 and 38 atoms.

Enzymatic reactions were carried out with templates NucT4 and T15 respectively (Figure 5). Polymerases used were *Taq* and *Chy*-Klenow polymerases at concentrations of 0,1 units per reaction. The derivative concentrations were 5 and 50 µM, and in reactions with 50 µM additionally 12,5 µM dATP and dGTP were added in an extra reaction tube, to monitor further elongation with natural dNTP's (lanes 8, 11, 14, 23, 26 and 29).

In this example, incorporation efficiency proves to depend on the linker length. *Taq* polymerase incorporates the 17-atom-spacer compound only two times, with a synthesis stop after only one incorporation on both templates (lanes 7-9 and 15, 16). No further reaction products were detected. Chy-Klenow-polymerase showed a maximum insertion of three (trace amounts of four insertions, lane 31) derivatives, but also with a serious stop after two incorporations, whereas the stop after only one insertion is drastically decreased (compare lanes 6, 7, 15 and 16 with lanes 21, 22, 30 and 31).

The intermediate length spacer compound (24 atoms) was polymerized into three positions by Taq-polymerase and into four positions with Chy-Klenow-polymerase (template NucT4, compare lanes 9-11 with lanes 24-26). The occurrence of additional bands in lane 11 may come from false incorporations of natural dNTP's present in the reaction mix. But these intermediate bands are lacking in lane 26 when *Chy*-polymerase is used. Here (lane 26) a further elongation after the template instructed polymerisation of four derivatized nucleotides is observed (decrease of stop-band after four incorporations, but a full length product could not be detected. Most probably synthesis stopped after position 10 (main product) and a minor product of one (additional) false incorporation in position 11 is detectable. With template NucT15 also additional polymerisation events for *Taq*-polymerase (five) and *Chy*-Klenow-polymerase (nine) are detectable (see lanes 17, 18 and 32, 33 respectively). The nature of intermediate bands occurring in lane 18 is not understood, because they are lacking in lanes 32 and 33, so that a contamination of uncoupled dUTP's (bearing only the spacer attached to the deoxynucleoside mojety, but not the dye) seems to be unlikely. So the intermediate length spacer compound is incorporated into more neighbouring positions, than the short, 17-atom-compound, but still trends to lead to synthesis-stops after 2 or more insertions.

The 38 atom spacer compound however, differs from the other two (17 and 24 atom distance) substances in its ability to be incorporated. First, with template NucT4 by far less synthesis stops are detectable during incorporations 1-4 with both polymerases used (see lanes 12- 14 and 27, 29; in lane 28 obviously addition of polymerase was omitted, as no elongation at all could be detected). The natural stops at position 4 however, could be overcome by addition of dATP and dGTP. In lane 14 (*Taq*-polymerase) the dissappearance of the stop in position 4 is seen, followed by the incorporation of the natural dATP and dGTP, followed by further incorporation of two modified nucleotides, with a main product ending at position 7 of the template and a minor product, ending at position 8. Lane 29 demonstrates, that also *Chy*-polymerase was able to elongate the four incorporated Cy-5-dUMP's, but far less effective, than it was catalyzed by *Taq*-polymerase. On the other hand, the product length is higher with *Chy*-polymerase (a total of 10 out of 12 incorporations is documented).

With template NucT15 *Taq*-polymerase was able to synthesize stretches of 7 (lane19) and 6 (lane 20) Cy-5-Uracil-derivatives, with major products of 5 and 6 incorporations. *Taq*-Polymerase produced a lot of synthesis-stops, that were not present in the respective reactions with *Chy*-polymerase (lanes 34 and 35). This enzyme, additionally, was able to polymerize 8 incorporations, with major products between 5 and 7 Cy-5-dU's in line, without any detectable stops below 4 insertions. So one can conclude, that the spacer arm length is a critical value for efficient incorporation. In this case a trend towards longer spacer compounds (24- and 38-atoms) resulted in more effective insertions and less synthesis stops during polymerisation. But there also are some differences between the polymerases used. In this example a truncated form of an A-type polymerase (*Chy*-Klenow-fragment), showed a better incorporation performance, than did *Taq* wild type polymerase.

### Example 8:

### Incorporation of Cy-dUTP and MR121-dUTP with different linker-arm lengths by a B-type polymerase.

In this example the incorporation of the three Cy5-dUTP of example 4 and additionally three MR121-dUTP derivatives with 8, 13 and 24 -atoms spacer-arms by *Tgo* exo⁻-polymerase (B-type, 0,1 unit per reaction) was examined. The templates used to examine polymerisation were again NucT4 and T15 (Figure 6).

Using template NucT4, without addition of dATP and dGTP the polymerisation stopped for all three Cy-5-variants after the template-instructed four incorporations (lanes 6, 7, 9, 10, 12, 13). A comparison of these lanes also indicates, that the four incorporations of, e.g. the smallest (17-atom-spacer) compound migrated in a gel-position of about 13 regular nucleotide incorporations (lane 3 of the control). The Cy-5-compounds with longer spacers were shifted relative to this band, due to their higher molar masses. Another remarkable feature is, that here, as it was the case in example 4, the short spacer arms led to stops during synthesis after 1, 2 and 3 incorporations. The 24-atom spacer produced a significantly different pattern. Here (lane 9), only at low triphosphate concentrations (5 µM), a single stop at position 3 is detectable. The 38-spacer derivative on the other hand was incorporated without any significant stop (lanes 12-14). All primers were further elongable from this fourth position by adding the required natural dATP and dGTP, giving rise to the full length products in the case of the 17- and 24-atom compounds. The 38-atom spacer compound could however only be incorporated, together with the other two nucleotides until the tenth polymerisation step.

The use of template NucT15 revealed a similar trend concerning the incorporation, as seen with NucT4. The short linker compound was introduced up to 10 times into nascent DNA, with major stops seen after 2, 3, 4 incorporations, and a continuum of synthesis breaks up to 10 incorporations (lanes 24, 25). The intermediate linker compound, (lanes 26, 27) showed one clear stop after three incorporations at low (suboptimal) derivative concentrations (5 µM, lane 26), which could be overcome with higher triphosphate concentrations (lane 27). With this compound also 10 neighbouring polymerisation steps were detectable, which is roughly the amount of one helix-turn of B-DNA.

A significant better incorporation was achieved with the 38 -atom spacer nucleotide. This derivative was introduced in 11 of 18 possible positions (lanes 28, 29), but the stops during synthesis occurred to a far lesser extent. Almost no stops were detectable at high concentrations (50 µM) below 7 incorporations (lane 29). The main products of this reaction range between 8 and 10 incorporations.

A similar trend, concerning the incorporation of the three MR121-dUTP deoxynucleosidetriphosphates is seen in lanes 15-23 and 30-35. Here the short linker arms (8 atoms) led to synthesis stops after 2, 3 and 4 incorporations (lanes15-20) with template NucT4, and at low concentrations (5 µM) also with template NucT15 (lane 30). The two nucleotides with the longer spacer molecules (13 and 24 atoms) showed no stop bands, but also no homogenous products, only diffuse high molecular smear in gels (lanes 20-23). Discrete stop synthesis bands are not visible. The primer on the other hand, is almost completely consumed (compare with control lanes 1, 2, 4 and 5), which indicates, that it has been elongated by the DNA polymerase used in combination with the respective derivatized nucleotide. An explanation for this fact is probably, that the newly incorporated dye-tagged nucleotides changed the physico-chemical properties of the DNA in that way, that the resulting molecules could not be analyzed with conventional DNA analytic methods.

In figure 6a a part of the blot of figure 6 (lanes 6-23) was analyzed with the Storm imager (Molecular Dynamics). It is shown, that in principal the same banding pattern in comparision with fig. 6 could be detected. But here only products beginning with at least one incorporated dye molecule are detectable (lanes 1-9). As expected, the non elongated Dig-labeled primer produces no signal. The MR121-tagged deoxynucleotides gave only a poor fluorescence signal, which fits together with the non optimal excitation and emission parameters of MR121 in this detection system. But similar, as in the chemiluminescence detection system, also here no discrete product bands could be analyzed for spacerlengths of more than 8 atoms (compare lanes 12, 13 with lanes 15-18).

In this example it is demonstrated, that a B-type polymerase with a mutated 3'-5'-exonuclease function incorporated the Cy-5-dUTP compounds better than the tested A-type polymerases of example 4. The trend of a better incorporation, the longer the spacer molecules are, is verified for A and also B-type enzymes respectively. The very hydrophobic fluorescent dye MR 121 altered the properties of the DNA after incorporation, that these molecules could not be analyzed with this experimental setup.

### Example 9:

### Synthesis of a DNA with all four natural occurring dNTP's replaced by derivatized compounds.

This experiment was carried out with templates cass 1, cass 5 and cass 10 (Figure 7). The polymerases used were *Tgo* exo- and *Vent* exo⁻ (0,1,0,5 and 1u respectively). Natural dNTP's were completely replaced by their derivatized analogues except in control reactions (lanes 1-6). Products with full reaction mixes and templates cass 1, cass 5 and cass 10 are shown in lane 3, 4 and 5, corresponding to 4+1, 20+1 and 40+1 nucleotide incorporations. The terminal addition of one "extra" nucleotide is a commonly known feature of *Taq* polymerase [2].

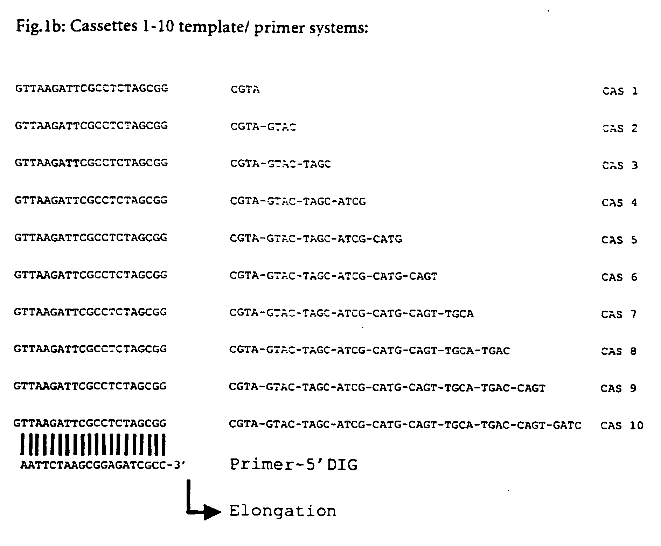

In lanes 7-10 the reaction products with cass 1 and *Vent* exo⁻ polymerase are shown. Reaction 7 (lane 7) contained Hex⁷c⁷G_{d} being the exclusive nucleotide triphosphate present in the assay. Here the primer was elongated exactly by one nucleotide, corresponding to the base pairing rules (see fig. 1b: sequences of Cass 1-10 in materials and methods), no false incorporation was detectable. Reaction 8 (lane 8) contained additionally Rosamin-dCTP (and no other nucleotides). It can be seen, that the stop after the incorporation was overcome, and another (shifted) band appeared in the range of 5-6 incorporated natural dNTP's. This means, the terminal Hex⁷-c⁷G_{d}-residue from reaction 7 has been further elongated by Rosamin-dCTP. In lane 9 the reaction mixture contained additionally to reaction 8 Hex⁷c⁷A_{d}. Again the stop of reaction 8 vanished and was elongated by the newly added compound (note the small migration distance to the product of reaction 8, which is in good agree with the comparatively minor modification of the 7-deaza-dATP). But here instead of a clearly defined single band a total of three bands is visible, which indicates false incorporations, most probably opposite to the Adenine-residue, the last base in the template sequence. In reaction 10 additionally to the other three nucleotides of reaction 9, Biotin-16-dUTP was added. Here the proper nucleotide was inserted opposite to A in the template sequence. Band splitting was also here detectable, as a consequence of stereoisomer occurrence as mentioned before (Mühlegger, pers. communication) The misincorporations were detectable only to a small extent.

In lanes 11-14 the same reactions, as in lanes 7-10 are documented, now catalyzed by *Tgo* exo⁻ polymerase. A remarkable feature of this enzyme is the fidelity, because the misincorporations of *Vent* exo⁻ (lane 9) did not occur with this polymerase (see lane 13). Here only one discrete band is visible after adding Hex⁷c⁷A_{d} to the reaction mixture. From this position Biotin-16-dUTP was attached to give raise to the final product.

In lanes 15-22 the same experiments as in lanes 7-14, but with template cass 5 are shown. Here similar reaction product patterns are obtained, when the two polymerases are compared (lanes *15-18: Vent* exo- polymerase; lanes 19-22: *Tgo* exo⁻ polymerase). But the products of the complete reaction mixes (lanes 18 and 22) could not be analyzed properly concerning their length. This was also not possible, when higher concentrations of nucleotides (50 µM) and 0,5 units instead of 0,1 units of polymerase were used (lanes 23-26). Here the tendency of false incorporations was enhanced, compared with the reactions with low nucleotide and polymerase concentrations (compare lanes 16, 17 with lanes 24, 25). Especially in lane 25 and also with cass 10, (lane 29) a large amount of different intermediate products can be determined (compare with lane 13) when an incomplete nucleotide mix is used. But all of these misincorporations are obviously not formed, when the complete dNTP mix is used (see lanes 22, 26, 30), because the abortive synthesis products are missing here.

When cass 10 serves as template, longer reaction products are synthesized, than with cass 5, but a defined product is not produced (lanes 30, 32). There are several intermediate products detectable. No information is obtained, whether the target sequence has been reached. A tendency is observable, that *Tgo* exo⁻ polymerase produces less stops and longer products (compare lane 30 with lane 32). If Rosamin-dCTP is replaced by regular dCTP (lanes 34-36), a putative final product is polymerized (lane 36). Here the stops occur to a much lesser extent than in reactions 30 and 32. As the final product of reaction 36 migrates in a higher molecular weight position, than do the longest products of reactions 30 and 32, although the derivatized Rosamin-dGTP was replaced by the regular dGTP, which has a lower molecular weight, it is very likely, that in reactions 30 and 32 full length synthesis failed, whereas it is obvious, that in reaction 36 target sequence is reached.

In this example only with cass 1 the full replacement of all four nucleotides by derivatives is documented (lanes 7-14).

The bands migrating in the range of 18 incorporated natural nucleotides (lanes 15-26, when cass 5 is used) and in the range of 38-39 natural nucleotides, when cass 10 is used (lanes27-30 and 32-36) correspond to not completely denatured template primer heteroduplexes, which can be detected also in some other control experiments (data not shown).

### Example 10:

### Complete replacement of natural dNTP's by derivatized Nucleotide-triphosphates and polymerisation of a 40 base pair target sequence.

With the templates used in example 6, it was not possible to determine the product length of reactions with cass 5 and 10 precisely, due to synthesis stops and irregular migration during electrophoresis. In this example (Figure 8) we used the complete set of templates cass 1- 10, in order to determine the actual number of polymerisation events catalyzed by the DNA polymerase. Cassettes 1-10 consist of 10 oligonucleotides, made of building blocks, which differ in their length from each other by 4 nucleotides. In these building blocks each base is represented once. The nucleotides used here are: Hex⁷c⁷G_{d}, Digoxigenin-dCTP, Aminopentinyl-7-deaza-dATP and Biotin-16-dUTP. Rosamin-dCTP was replaced in this assay by Dig-dCTP, because the latter did not interfere with the electrophoretic mobility of the resulting polymerisation products (data not shown). The polymerase used was *Tgo* exo⁻ polymerase (0.1 unit per reaction).

Reaction products with cass 1 could be visualized only after overexposure and are marked with asterisks in fig. 8. Control reactions with cass 10 as template and natural dNTP's (40 incorporations possible) are shown in lanes 1-4.

It is shown, that with the increase of template-length used (cass 1-10), also the length of polymerisation products increased. The number of synthesis stops is comparatively low, when compared with the results of example 6. If the In of the maximal number of nucleotides incorporated (4, 8, 12...) is plotted against the increment of altered electrophoretic mobility (distance from primer to product) from cassette to cassette, one can see a linear correlation (fig. 8a). This is strongly indicative, that full length products have been synthesized with all cassette-templates. Here a DNA-sequence has been built up enzymatically (maximal incorporated Nucleotides: 40, each base 10 times), consisting of four derivatized nucleotides, whereby the purines are replaced by their 7-deaza-analogues, and the pyrimidines being the regular nucleobases attached to a reporter group.

### Example 11:

### Incorporation of modified nucleotides by polymerase chain reaction (PCR).

As a second experimental method for measuring effective modified dNTP incorporation, PCR was chosen. In PCR, unlike at the primer extension reactions, the labelled nucleotides after a few rounds of amplification are also present in the template strand, so that in the end both strands consist of derivatized compounds, with the exception of the unlabeled primer moieties at each 5' end of duplex DNA. In the reaction mixtures the respective natural nucleotides were completely replaced by their labelled counterparts. Amplification parameters were:
1x 95°C 2 min
35x 95°C 30 sec; 60°C 30 sec, 72°C 60 sec.
Template concentration was 0,5-0,7 fmol linearized plasmid containing the tPA-gene per reaction; Primer (tPa Exon9 5'-TGG.TGC.CAC.GTG.CTG.AAG.AA-3' and tPA Exon 12 5'-AGC.CGG.AGA.GCT.CAC.ACT.C-3', giving rise to a 517 bp DNA fragment and tPA Exon 10 5'-AGA.CAG.TAC.AGC.CAG.CCT.CA-3' and tPA Exon 11 5'-GAC.TTC.AAA-TTT.CTG.CTC.CTC-3', giving rise to a 264 bp DNA product) were 20 pmoles each.

Polymerase amount was 2.6 units per reaction and nucleotide concentration was 200µM each dNTP. After the cycling procedure the probes were analyzed on 2% Agarose gels. A parameter for good acceptance of a given derivative was the building of a product band.

In general these products were shifted in the molecular mass due to the higher molecular weight of the monomeric building blocks. The shift towards higher molmasses was strictly dependent on the compound used. In the example shown in Fig. 9, the biotinylated 264bp product migrated with an apparent molecular weight/ mass of about 520bp. This fragment was isolated from the gel (lane D), the DNA recovered and used as template in a new round of amplification with natural dNTP's, giving rise to the original 264bp product (reversion). When the DNA template of lane D was dilutet sequentially by a factor of 10 for each dilution step (lanes E-J) a decrease in product yield was seen (template titration). As the template was recovered from a gel position around 500 bp, it is very unlikely, that residual plasmid DNA of the linearisized plasminogen activator-gene containing plasmid served as template here. The reverted product was then purified and sequenced. The determination of the sequence revealed no alteration in sequence, compared with the original Plasmid.

Table 2 shows, that several polymerases were able to synthesize products with different derivatized deoxynucleotides.

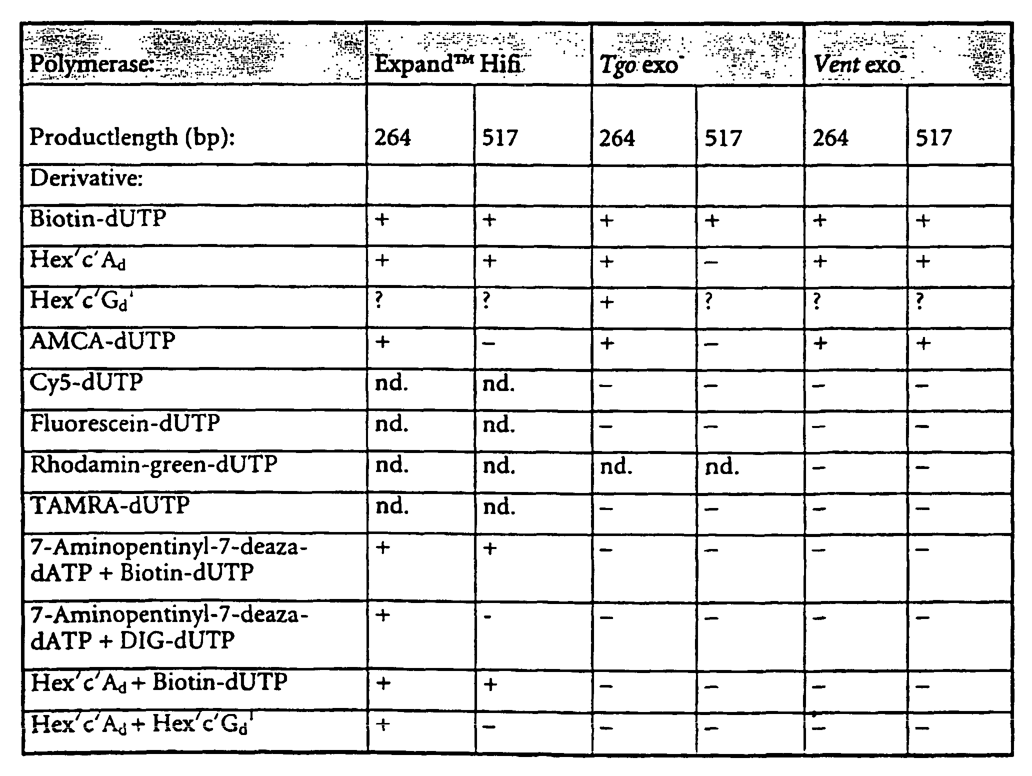

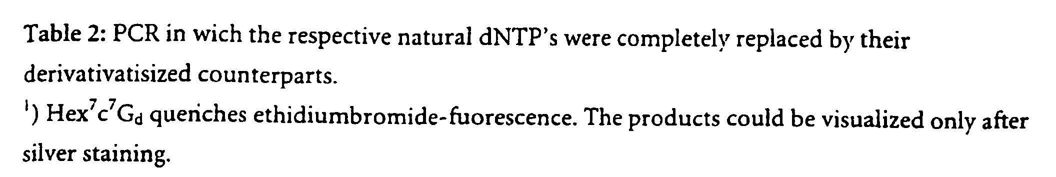

It should be noted, that in the case of Aminopentinyl-7-deaza-dATP by several polymerases only in combination with other derivatives a product has been formed. When the dTTP was also replaced by (the A-T-base-pair being completely replaced), Dig- or Biotin-16-dUTP, then a product was formed. This product has now introduced an aminofunctionalyzed base, which makes it an ideal substrate for "post labeling" with either fluorescent dyes or other detectable groups.

### Appendix:

### References:

[1] Perler, F. B.;Kumar,S.,Kong, H. (1996) Thermostable DNA polymerases; *Advances in protein chemistry,* Vol. 48, 377-435
[2] Hu G (1993) DNA polymerase-catalyzed addition of nontemplated extra nucleotides to the 3' end of a DNA fragment; *DNA & Cell Biol.* 12(8):763-70.
[3] Braithwaite DK, Ito (1993) Compilation, alignment, and phylogenetic relationships of DNA polymerases. *Nucleic Acids Res.* 21(4):787-802
[4] *Nonradioactive In Situ Hybridization Application Manual* 2nd edition, Boehringer Mannheim GmbH (1996).
[5] Jett J. H. et al. (1995) US Patent 5,405,747: Method for rapid base sequencing in DNA and RNA with two base labeling.
[6] Jett JH, Keller RA, Martin JC, Marrone BL, Moyzis RK, Ratliff RL, Seitzinger NK, Shera EB, Stewart CC (1989); High-speed DNA sequencing: an approach based upon fluorescence detection of singlemolecules. *J Biomol Struct Dyn* 7(2):301-309.
[7] Hiyoshi M, Hosoi S (1994); Assay of DNA denaturation by polymerase chain reaction-driven fluorescent label incorporation and fluorescence resonance energy transfer. *Anal* Biochem 221(2):306-311
[8] Yu H, Chao J, Patek D, Mujumdar R, Mujumdar S, Waggoner AS (1994); Cyanine dye dUTP analogs for enzymatic labeling of DNA *probes.Nucleic Acids* Res. 22(15):3226-32.
[9] Starke HR, Yan JY, Zhang JZ, Muhlegger K, Effgen K, Dovichi NJ (1994); Internal fluorescence labeling with fluorescent deoxynucleotides in two-label peak-height encoded DNA sequencing by capillary electrophoresis. *Nucleic Acids Res.* 22(19):3997-4001.
[10] Davis LM, Fairfield FR, Harger CA, Jett JH, Keller RA, Hahn JH, Krakowski LA, Marrone BL, Martin JC, Nutter HL, et al (1991); Rapid DNA sequencing based upon single molecule detection. *Genet Anal Tech Appl* 8(1):1-7.
[11] Goodman, M. F. and Reha-Krantz L. Patent: (International Application Number PCT/US97/06493, WO 97/39150, Synthesis of Fluorophore-labeled DNA.

## Claims

1. A method for enzymatic nucleic acid labeling which uses a DNA polymerase, a nucleic acid template, a primer and modified nucleoside triphosphates which can be incorporated by the polymerase into the newly synthesized DNA wherein the DNA Polymerase is a genetically engineered exo-minus mutant of B-type polymerases not exhibiting 3' exonoclease activity and at least two natural deoxynucleoside triphosphates are replaced entirely by the corresponding modified deoxynucleoside triphosphates or derivatives thereof such that in the nucleic acid generated, two of the four bases carry modifications and full target length is achieved.

2. The method of claim 1 in which the modified nucleotides incorporated into the newly synthesized nucleic acid are detectable by nonradioactive methods.

3. The method of claims 1-2 in which enzymatic nucleic acid labeling is performed in a reaction in which three natural deoxynucleoside triphosphates are replaced entirely by the corresponding modified deoxynucleoside triphosphates or derivatives thereof such that in the synthesized nucleic acid three of the four bases carry modifications and full target length is achieved.

4. The method of claim 1-2 in which enzymatic nucleic acid labeling is performed in a reaction in which four natural deoxynucleoside triphosphates are replaced entirely by the corresponding modified deoxynucleoside triphosphates or derivatives thereof such that in the synthesized nucleic acid consists entirely of modified bases and full target length is achieved.

5. The method of claim 4 in which all four bases carry different labels.

6. The method of claim 1-5 in which the labeling of one nucleic acid strand is performed with one primer used.

7. The method of claim 1-6 in which the labeling of double stranded nucleic acid is performed in an amplification reaction wherein two or more or degenerate or Inosine containing primers are used.

8. The method of claim 1-7 in which the enzymes used are a reverse transcriptase and a B-type DNA polymerase.

9. The method of claim 1-8 wherein the modified nudeotides are attached to hydrophilic dyes or hydrophilic labels.

10. The method of claim 1-9 wherein the B-type polymerase exhibits no 3'exonuclease activity and wherein the hydrophilic label is selected from the group consisting of carbocyanines, rhodamines, fluoresceins, cumarines, vitamines as biotin, haptens as digoxigenine, oxazines and hormones as cholesterol and estradiaol and wherein the linker which attaches the dye to the nucleotide has a length of about 15 carbon atoms or more.

11. The method of claim 1-10 which is used for DNA synthesis and simultaneous DNA labeling by PCR.

## Patentansprüche

1. Verfahren zur enzymatischen Nukleinsäuremarkierung, bei der eine DNA-Polymerase, eine Nukleinsäurematrize, ein Primer und modifizierte Nukleosidtriphosphate, die durch die Polymerase in die neu synthetisierte DNA eingebaut werden können, verwendet werden,
worin die DNA-Polymerase eine genetisch manipulierte Exominus-Mutante von Polymerasen des B-Typs, die keine 3'-Exonukleaseaktivitität zeigen, ist und mindestens zwei natürliche Desoxynukleosidtriphosphate ganz durch die entsprechenden modifizierten Desoxynukleosidtriphosphate oder Derivate davon in der Weise ersetzt werden, dass in der erzeugten Nukleinsäure zwei der vier Basen Modifikationen tragen und die volle Targetlänge erreicht wird.

2. Verfahren nach Anspruch 1,
worin die in die neu synthetisierte Nukleinsäure eingebauten modifizierten Nukleotide durch nichtradioaktive Methoden nachweisbar sind.

3. Verfahren nach den Ansprüchen 1 bis 2,
worin die enzymatische Nukleinsäuremarkierung in einer Reaktion durchgeführt wird, in der drei natürliche Desoxynukleosidtriphosphate ganz durch die entsprechenden modifizierten Desoxynukleosidtriphosphate oder Derivate davon in der Weise ersetzt werden, dass in der synthetisierten Nukleinsäure drei der vier Basen Modifikationen tragen und die volle Targetlänge erreicht wird.

4. Verfahren nach den Ansprüchen 1 bis 2,
worin die enzymatische Nukleinsäuremarkierung in einer Reaktion durchgeführt wird, in der vier natürliche Desoxynukleosidtriphosphate ganz durch die entsprechenden modifizierten Desoxynukleosidtriphosphate oder Derivate davon in der Weise ersetzt werden, dass die synthetisierte Nukleinsäure ganz aus modifizierten Basen besteht und die volle Targetlänge erreicht wird.

5. Verfahren nach Anspruch 4,
worin alle vier Basen verschiedene Markierungen tragen.

6. Verfahren nach den Ansprüchen 1 bis 5,
worin die Markierung eines Nukleinsäurestranges mit einem verwendeten Primer durchgeführt wird.

7. Verfahren nach den Ansprüchen 1 bis 6,
worin die Markierung der doppelsträngigen Nukleinsäure in einer Amplifikationsreaktion durchgeführt wird, wobei zwei oder mehrere oder degenerierte Primer oder Inosin-enthaltende Primer verwendet werden.

8. Verfahren nach den Ansprüchen 1 bis 7,
worin die verwendeten Enzyme eine reverse Transcriptase und eine DNA-Polymerase vom B-Typ sind.

9. Verfahren nach Anspruch 1 bis 8,
worin die modifizierten Nukleotide an hydrophile Farbstoffe oder hydrophile Markierungen gebunden sind.

10. Verfahren nach den Ansprüchen 1 bis 9,
worin die Polymerase vom B-Typ keine 3'-Exonukleaseaktivität zeigt und worin die hydrophile Markierung ausgewählt ist aus der Gruppe, die aus Carbocyaninen, Rhodaminen, Fluoreszeinen, Cumarinen, Vitaminen, wie Biotin, Haptenen, wie Digoxigenin, Oxazinen und Hormonen, wie Cholesterin und Östrogen besteht und worin der Linker, der den Farbstoff an das Nukleotid bindet, eine Länge von etwa 15 Kohlenstoffatomen oder mehr aufweist.

11. Verfahren nach den Ansprüchen 1 bis 10,
das für die DNA-Synthese und gleichzeitige DNA-Markierung durch PCR angewendet wird.

## Revendications

1. Procédé pour le marquage enzymatique d'un acide nucléique qui utilise une ADN polymérase, une matrice d'acide nucléique, une amorce et des nucléosides triphosphates modifiés qui peuvent être incorporés par la polymérase dans l'ADN nouvellement synthétisé, dans lequel l'ADN polymérase est un mutant exo-moins de polymérases de type B, produit par génie génétique, n e manifestant pas d'activité 3'-exonucléase et au moins deux désoxynucléosides triphosphates naturels sont entièrement remplacés par les désoxynucléosides triphosphates modifiés correspondants ou leurs dérivés de telle sorte que, dans l'acide nucléique produit, deux des quatre bases portent des modifications et que toute la longueur de la cible est atteinte.

2. Procédé selon la revendication 1, dans lequel les nucléotides modifiés incorporés dans l'acide nucléique nouvellement synthétisé sont détectables par des procédés non radioactifs.

3. Procédé selon les revendications 1 et 2, dans lequel le marquage enzymatique d'un acide nucléique est réalisé dans une réaction dans laquelle trois désoxynucléosides triphosphates naturels sont entièrement remplacés par les désoxynucléosides triphosphates modifiés correspondants ou leurs dérivés de telle sorte que, dans l'acide nucléique synthétisé, trois des quatre bases portent des modifications et que toute la longueur de la cible est atteinte.

4. Procédé selon les revendications 1 et 2, dans lequel le marquage enzymatique d'un acide nucléique est réalisé dans une réaction dans laquelle quatre désoxynucléosides triphosphates naturels sont entièrement remplacés par les désoxynucléosides triphosphates modifiés correspondants ou leurs dérivés de telle sorte que l'acide nucléique synthétisé est entièrement constitué par des bases modifiées et que toute la longueur de la cible est atteinte.

5. Procédé selon la revendication 4, dans lequel les quatre bases portent des marqueurs différents.

6. Procédé selon les revendications 1 à 5, dans lequel le marquage d'un brin de l'acide nucléique est effectué avec l'utilisation d'une amorce.

7. Procédé selon les revendications 1 à 6, dans lequel le marquage d'un acide nucléique en double brin est effectué dans une réaction d'amplification, et dans lequel sont utilisées deux ou plusieurs amorces ou bien des amorces dégénérées ou contenant de l'Inosine.

8. Procédé selon les revendications 1 à 7, dans lequel les enzymes utilisées sont une transcriptase inverse et une ADN polymérase de type B.

9. Procédé selon les revendications 1 à 8, dans lequel les nucléotides modifiés sont attachés à des colorants hydrophiles ou à des marqueurs hydrophiles.

10. Procédé selon les revendications 1 à 9, dans lequel la polymérase de type B ne manifeste aucune activité 3'-exonucléase et dans lequel le marqueur hydrophile est choisi dans le groupe constitué par des carbocyanines, des rhodamines, des fluorescéines, des coumarines, des vitamines comme la biotine, des haptènes comme la digoxigénine, des oxazines et des hormones comme le cholestérol et l'oestradiol, et dans lequel le liant qui attache le colorant au nucléotide a une longueur d'environ 15 atomes de carbone ou davantage.

11. Procédé selon les revendications 1 à 10 qui est utilisé pour la synthèse d'un ADN et le marquage simultané de l'ADN par PCR.
